# NEW EUROPEAN PATENT SPECIFICATION

(11) **EP 3 169 797 B2**
(45) Date of publication and mention of the opposition decision: **19.02.2025**
(45) Mention of the grant of the patent: 21.08.2019
(21) Application number: 15734713.9
(22) Date of filing: 13.07.2015
(51) Int. Cl.: C12Q 1/6886

(54) **MEANS AND METHODS FOR IDENTIFYING A PATIENT HAVING A BRAF-POSITIVE CANCER AS A NON-RESPONDER TO A BRAF INHIBITOR AND AS A RESPONDER TO AN MAPK/ERK INHIBITOR**
MITTEL UND VERFAHREN ZUR IDENTIFIZIERUNG EINES PATIENTEN MIT EINEM BRAF-POSITIVEN KREBS ALS NON-RESPONDER AUF EINEN BRAF-INHIBITOR UND ALS RESPONDER AUF EINEN MAPK/ERK-INHIBITOR
MOYENS ET PROCÉDÉS PERMETTANT D'IDENTIFIER UN PATIENT AYANT UN CANCER BRAF-POSITIF COMME ÉTANT NON-RÉPONDEUR À UN INHIBITEUR DE BRAF ET RÉPONDEUR À UN INHIBITEUR MAPK/ERK

(30) Priority: 14.07.2014 EP 14176944
(43) Date of publication of application: 24.05.2017
(73) Proprietor: Universität Zürich Prorektorat MNW, 8006 Zürich (CH)
(72) Inventor: LEVESQUE, Mitchell Paul, CH-8712 Stäfa (CH); DUMMER, Reinhard, CH-8044 Zurich (CH); WIDMER, Daniel, CH-8057 Zürich (CH); RAAIJMAKERS, Marieke Ineke Geertje, CH-8037 Zürich (CH)
(74) Representative: Altmann Stößel Dick Patentanwälte PartG mbB
(86) International application number: PCT/EP2015/065986
(87) International publication number: WO 2016/008853

(56) References cited:
- WO-A1-2012/068468
- WO-A2-2012/058532
- WO-A2-2012/068562
- WO-A2-2014/052613
- US-A1- 2014 018 372
- US-A1- 2014 018 405
- JAYKUMAR THUMAR ET AL: "MEK targeting in N-RAS mutated metastatic melanoma", MOLECULAR CANCER, BIOMED CENTRAL, LONDON, GB, vol. 13, no. 1, 4 March 2014 (2014-03-04), pages 45, XP021178565, ISSN: 1476-4598, DOI: 10.1186/1476-4598-13-45
- J. G. GREGER ET AL: "Combinations of BRAF, MEK, and PI3K/mTOR Inhibitors Overcome Acquired Resistance to the BRAF Inhibitor GSK2118436 Dabrafenib, Mediated by NRAS or MEK Mutations", MOLECULAR CANCER THERAPEUTICS, vol. 11, no. 4, 1 April 2012 (2012-04-01), pages 909 - 920, XP055057160, ISSN: 1535-7163, DOI: 10.1158/1535-7163.MCT-11-0989
- E. ROMANO ET AL: "Identification of Multiple Mechanisms of Resistance to Vemurafenib in a Patient with BRAFV600E-Mutated Cutaneous Melanoma Successfully Rechallenged after Progression", CLINICAL CANCER RESEARCH, vol. 19, no. 20, 15 August 2013 (2013-08-15), pages 5749 - 5757, XP055156251, ISSN: 1078-0432, DOI: 10.1158/1078-0432.CCR-13-0661
- RAMIN NAZARIAN ET AL: "Melanomas acquire resistance toB-RAF(V600E) inhibition by RTK or N-RAS upregulation", NATURE, NATURE PUBLISHING GROUP, UNITED KINGDOM, vol. 468, no. 7326, 16 December 2010 (2010-12-16), pages 973 - 979, XP002633500, ISSN: 0028-0836, DOI: 10.1038/NATURE09626
- F. M. KAPLAN ET AL: "SHOC2 and CRAF Mediate ERK1/2 Reactivation in Mutant NRAS-mediated Resistance to RAF Inhibitor", JOURNAL OF BIOLOGICAL CHEMISTRY, vol. 287, no. 50, 17 October 2012 (2012-10-17), pages 41797 - 41807, XP055156287, ISSN: 0021-9258, DOI: 10.1074/jbc.M112.390906
- M SENSI ET AL: "Mutually exclusive NRASQ61R and BRAFV600E mutations at the single-cell level in the same human melanoma", ONCOGENE, vol. 25, no. 24, 6 February 2006 (2006-02-06), pages 3357 - 3364, XP055127101, ISSN: 0950-9232, DOI: 10.1038/sj.onc.1209379

## Description

The present invention relates to the field of diagnostics, in particular, cancer diagnostics. More specifically, it relates to a method for identifying whether a subject suffering from a BRAF-positive cancer is a non-responder to a BRAF inhibitor, or not, and/or is a responder to an MAPK/ERK inhibitor, a method for assessing responsiveness to targeted therapy in a subject and a method for assessing cancer in a subject. Moreover, contemplated by the invention are a kit and a device for diagnosing cancer. Further, the invention relates to a MAPK/ERK inhibitor for use in treating a subject suffering from a BRAF-positive cancer.

Melanoma therapies for advanced disease have made great progress in the last few years ¹⁻³, but primary intrinsic resistance of some patients to targeted therapy, as well as the onset of delayed acquired resistance in most other patients, continue to pose a major challenge for the clinical management of metastatic melanoma ⁴.

However, the advent of next generation sequencing (NGS) technologies allows addressing the question of how conventional therapies influence the heterogeneous landscape of genetic variations within patients and to identify the source of therapeutic resistance. Aside from elucidating new mechanisms of cancer progression, NGS applications also provide large datasets for the quantification and modeling of clonal diversity changes over time. In some cancers, global genetic diversity metrics have been shown to be predictive of neoplastic progression ⁵.

Metastatic melanoma, in particular, has one of the highest mutation rates of any cancer ⁶. Some studies have identified genomic characters such as the loss of heterozygosity that vary between primary tumors and metastases ⁷, and others have shown that this genetic heterogeneity is also present within individual tumors ⁸.

Within the context of therapeutic resistance, many genetic and transcriptional mechanisms of response to targeted therapy have recently been demonstrated across large patient cohorts, but the evolution of individual cancer genomes to systemic therapy remains poorly understood ^{9,10}. Minor subclones have been shown to exhibit decreased sensitivity to therapy ⁷, and more recent studies have revealed that patients receiving targeted BRAF inhibitors have diverse mechanisms of resistance arising from this underlying intra-tumoral molecular heterogeneity ¹¹.

Generally two different treatment resistance mechanisms can be distinguished: intrinsic (primary) and acquired (secondary). Intrinsically resistant tumors either do not initially respond or include a resistant subclone, which is rapidly selected during treatment, resulting in a failure to reduce tumor burden and rapid relapse. Acquired resistance mechanisms arise during treatment and may include selection or occurrence of additional activating mutations in genes of the MAPK pathway ^{10,24,25} or inactivating mutations in MAPK inhibitors ²⁶. Also, alternative splicing of the BRAF transcript and other non-genetic mechanisms have been reported to play a role in therapeutic resistance ²⁷. Despite a high number of studies dealing with this problem, the list of known resistance mechanisms is far from complete and in many individual cases, the mechanism of resistance remains unknown.

Activating BRAF or NRAS mutations are frequently found in human melanomas ^{10, 45-55}. Typically, the analyzed melanoma cells have a mutation in either BRAF or NRAS, but not a double mutation of BRAF and NRAS. As examples, the cell lines YUSAC (BRAF mutant V600E, NRAS wt), YUGEN (BRAF mutant V600E, NRAS wt) and YUDOSO (NRAS mutant Q61K, BRAF wt) disclosed in ⁴⁶ could be mentioned here. Although NRAS and BRAF activating mutations can coexist in the same melanoma, they are thought to be mutually exclusive at the single-cell level ⁴⁵. In addition, the presence of an NRAS mutation, for example NRASQ61R, or of a BRAF mutation, for example, BRAFV600E, is associated with distinct in vitro and in vivo growth properties and may directly impact the clinical management of the mutant melanoma ⁴⁵.
In light of the aforementioned tumor resistance mechanisms, it would be highly desirable to characterize cancers for suitable therapeutic interventions and, in particular, with respect to their capability to respond to BRAF inhibitor therapy.

The technical problem underlying the present invention can be seen as the provision of means and methods for complying with the aforementioned needs. The technical problem is solved by the embodiments characterized in the claims and herein below.

The present invention, thus, relates to a method for identifying whether a subject suffering from a BRAF-positive cancer, wherein said BRAF-positive cancer is comprised of a cell population derived from a single cell clone, is a non-responder to a BRAF inhibitor, or not, and/or is a responder to an MAPK/ERK inhibitor comprising the steps of:
(a) determining the presence or absence of at least one mutation in at least the NRAS gene in a sample of the subject, wherein said sample is derived from a single cell clone; and
(b) identifying the subject as a non-responder to a BRAF inhibitor and a responder to a MAPK/ERK inhibitor if the at least one mutation in the NRAS gene has been determined.

The method of the present invention, preferably, is an ex vivo method. Moreover, it may comprise steps in addition to those explicitly mentioned above. For example, further steps may relate to sample pre-treatments or evaluation of the results obtained by the method. The method may be carried out manually or assisted by automation. Preferably, step (a), and/or (b) may in total or in part be assisted by automation, e.g., by a suitable robotic and sensory equipment for the determination in step (a) and/or a computer-implemented calculation algorithm on a data processing device for the identification in step (b).

The term "identifying" as used herein means assessing whether the subject is a non-responder, or not, or is a responder, or not, to a BRAF inhibitor. Accordingly, identifying may aim to rule-in a subject into the groups of non-responders or to rule-out it from said group. Likewise, identifying may aim to rule-in a subject into the group of responders to rule out it from said group. Moreover, identifying also encompasses assessing that the subject is a responder to a MAPK/ERK inhibitor. As will be understood by those skilled in the art, such an assessment is, usually, not intended to be correct for 100% of the subjects to be investigated. The term, however, requires that the assessment is correct for a certain portion of subjects (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

The term "subject" as used herein relates to animals, typically mammals, and, more typically, humans. The subject according to the present invention shall suffer from a BRAF-positive cancer.

A "BRAF-positive cancer" as used herein refers to a cancer that comprises cancer cells, typically, derived from a single cell clone, having an impairment of the BRAF activity.
Typically, the BRAF activity is increased resulting in an activation of, inter alia, the MAPK-pathway in said cells. More typically, BRAF activation is caused by at least one mutation in the BRAF gene resulting in, e.g., a constitutive active BRAF protein or a BRAF protein that can not be controlled any longer within a cell. Particular BRAF mutations that result in an activated BRAF protein are specified elsewhere herein. In an aspect, the subject may or may not have received a BRAF inhibitor treatment. Typical BRAF-positive cancers in accordance with the present invention are melanoma cancer, non-Hodgkin lymphoma cancer, colorectal cancer, papillary thyroid carcinoma cancer, non-small-cell lung carcinoma cancer, hairy cell leukemia or adenocarcinoma of the lung. More typically, it is melanoma cancer.

The term "BRAF inhibitor" refers to a molecule that is capable of interfering with BRAF activity. A BRAF inhibitor may be an anti-BRAF antibody that specifically binds to BRAF protein and inhibits its activity. Moreover, a BRAF inhibitor may be an inhibiting nucleic acid. Inhibiting nucleic acids may be aptamers that specifically bind to BRAF protein and inhibit its activity. Other inhibiting nucleic acids may bind to BRAF transcripts and inhibit the translation thereof or degrade them. Typically, such inhibiting nucleic acids may be antisense nucleic acids, morpholino oligonucleotides, inhibitory RNA molecules such as siRNAs or micro RNAs, or ribozymes.

Antisense nucleic acid molecules are, typically, RNA and comprise a nucleic acid sequence which is essentially or perfectly complementary to the target transcript. In an aspect, an antisense nucleic acid molecule essentially consists of a nucleic acid sequence being complementary to at least 100 contiguous nucleotides, more preferably, at least 200, at least 300, at least 400 or at least 500 contiguous nucleotides of the target transcript. How to generate and use antisense nucleic acid molecules is well known in the art (see, e.g., Weiss, B. (ed.): Antisense Oligodeoxynucleotides and Antisense RNA : Novel Pharmacological and Therapeutic Agents, CRC Press, Boca Raton, FL, 1997). Morpholino oligonucleotides are synthetic nucleic acid molecules having a length of 20 to 30 nucleotides and, typically 25 nucleotides.

Morpholinos bind to complementary sequences of target transcripts by standard nucleic acid base-pairing. They have standard nucleic acid bases which are bound to morpholine rings instead of desoxyribose rings and linked through phosphorodiamidate groups instead of phosphates (see, e.g., Summerton 1997, Antisense & Nucleic Acid Drug Development 7* (3): 187-95). Due to replacement of anionic phosphates with the uncharged phosphorodiamidate groups eliminates ionization in the usual physiological pH range, so morpholinos in organisms or cells are uncharged molecules. The entire backbone of a morpholino is made from these modified subunits. Unlike inhibitory small RNA molecules, morpholinos do not degrade their target RNA molecules. Rather, they sterically block binding to a target sequence within a RNA and simply getting in the way of molecules that might otherwise interact with the RNA (see, e.g., Summerton 1999, Biochimica et Biophysica Acta 1489 (1): 141-58).

Small interfering RNAs (siRNAs) are complementary to target RNAs encoding a gene of interest and diminish or abolish gene expression by RNA interference (RNAi). Similarly, micro RNAs comprise complementary RNA targeting sequences and also act via RNAi mechanisms. Without being bound by theory, RNAi is generally used to silence expression of a gene of interest by targeting mRNA. Briefly, the process of RNAi in the cell is initiated by double stranded RNAs (dsRNAs) which are cleaved by a ribonuclease, thus producing siRNA duplexes. The siRNA binds to another intracellular enzyme complex which is thereby activated to target whatever mRNA molecules are homologous (or complementary) to the siRNA sequence. The function of the complex is to target the homologous mRNA molecule through base pairing interactions between one of the siRNA strands and the target mRNA. The mRNA is then cleaved approximately 12 nucleotides from the 3' terminus of the siRNA and degraded. In this manner, specific mRNAs can be targeted and degraded, thereby resulting in a loss of protein expression from the targeted mRNA. A complementary nucleotide sequence as used herein refers to the region on the RNA strand that is complementary to an RNA transcript of a portion of the target gene. dsRNA refers to RNA having a duplex structure comprising two complementary and antiparallel nucleic acid strands. Not all nucleotides of a dsRNA necessarily exhibit complete Watson-Crick base pairs; the two RNA strands may be substantially complementary. The RNA strands forming the dsRNA may have the same or a different number of nucleotides, with the maximum number of base pairs being the number of nucleotides in the shortest strand of the dsRNA. Preferably, the dsRNA is no more than 49, more preferably less than 25, and most preferably between 19 and 23, nucleotides in length. dsRNAs of this length are particularly efficient in inhibiting the expression of the target gene using RNAi techniques. dsRNAs are subsequently degraded by a ribonuclease enzyme into short interfering RNAs (siRNAs). The complementary regions of the siRNA allow sufficient hybridization of the siRNA to the target RNA and thus mediate RNAi. In mammalian cells, siRNAs are approximately 21-25 nucleotides in length. The siRNA sequence needs to be of sufficient length to bring the siRNA and target RNA together through complementary base-pairing interactions. The siRNA used with the Tet expression system of the invention may be of varying lengths. The length of the siRNA is preferably greater than or equal to ten nucleotides and of sufficient length to stably interact with the target RNA; specifically 15-30 nucleotides; more specifically any integer between 15 and 30 nucleotides, most preferably 15, 16, 17, 18, 19, 20, 21, 22, 23, 24, 25, 26, 27, 28, 29, and 30. By sufficient length is meant an oligonucleotide of greater than or equal to 15 nucleotides that is of a length great enough to provide the intended function under the expected condition. By stably interact is meant interaction of the small interfering RNA with target nucleic acid (e.g., by forming hydrogen bonds with complementary nucleotides in the target under physiological conditions). Generally, such complementarity is 100% between the siRNA and the RNA target, but can be less if desired, preferably 91%, 92%, 93%, 94%, 95%, 96%, 97%, 98%, or 99%. For example, 19 bases out of 21 bases may be base-paired. In some instances, where selection between various allelic variants is desired, 100% complementary to the target gene is required in order to effectively discern the target sequence from the other allelic sequence. When selecting between allelic targets, choice of length is also an important factor because it is the other factor involved in the percent complementary and the ability to differentiate between allelic differences. Methods relating to the use of RNAi to silence genes in organisms, including C. elegans, Drosophila, plants, and mammals, are known in the art (see, for example, Fire 1998, Nature 391:806-811; Fire 1999, Trends Genet. 15, 358-363; Sharp 2001, Genes Dev. 15,485-490; Hammond 2001, Nature Rev. Genet. 2, 1110-1119; Tuschl 2001, Chem. Biochem. 2, 239-245; Hamilton 1999, Science 286, 950-952; Hammond 2000, Nature 404, 293-296; Zamore 2000, Cell 101, 25-33; Bernstein 2001, Nature 409, 363-366; Elbashir 2001, Genes Dev. 15, 188-200; WO 0129058; WO 09932619; and Elbashir 2001, Nature 411: 494-498).

Ribozymes are catalytic RNA molecules possessing a well defined tertiary structure that allows for catalyzing either the hydrolysis of one of their own phosphodiester bonds (self-cleaving ribozymes), or the hydrolysis of bonds in other RNAs, but they have also been found to catalyze the aminotransferase activity of the ribosome. The ribozymes envisaged in accordance with the present invention are, preferably, those which specifically hydrolyze the target transcripts. In particular, hammerhead ribozymes are preferred in accordance with the present invention. How to generate and use such ribozymes is well known in the art (see, e.g., Hean J, Weinberg MS (2008). "The Hammerhead Ribozyme Revisited: New Biological Insights for the Development of Therapeutic Agents and for Reverse Genomics Applications". In Morris KL. RNA and the Regulation of Gene Expression: A Hidden Layer of Complexity. Norfolk, England: Caister Academic Press).

Furthermore, BRAF inhibitors may be small molecules that bind to BRAF and inhibit its activity. Such small molecule inhibitors of BRAF can be obtained by well known screening procedures or molecular modelling approaches aiming to identify compounds that bind to the active site of the BRAF kinase domain. BAY43-9006, also known as Sorafenib or Nexavar, is a small molecule compound that inhibits BRAF activity via binding to the inactive form of the kinase domain and blocks the activation thereof. PLX4032, also known as Vemurafenib, is a BRAF inhibitor that anchors itself in the ATP binding pocket of the kinase domain and, thereby, blocks activity of the active enzyme. In an aspect, the BRAF inhibitor referred to herein is selected from the group consisting of: LGX818 (Encorafenib), PLX4032 (Vemurafenib), GSK2118436 (Dabrafenib), GDC-0879, and BAY43-9006 (Sorafenib). More typically, the BRAF inhibitor is LGX818 (Encorafenib), PLX4032 (Vemurafenib) or GSK2118436 (Dabrafenib).

The term "non-responder to a BRAF inhibitor" refers to a subject exhibiting a BRAF-positive cancer which upon administration of a BRAF inhibitor shows progression or no or insignificant amelioration or cure of the cancer or after a period of response to treatment develops acquired resistance to therapy.

The term "MAPK/ERK inhibitor" refers to a molecule that is capable of interfering with MAPK activity and, in particular, ERK activity. A MAPK/ERK inhibitor may be an anti- MAPK/ERK antibody that specifically binds to MAPK/ERK proteins and inhibits their activity. Moreover, a MAPK/ERK inhibitor may be an inhibiting nucleic acid. Inhibiting nucleic acids may be aptamers that specifically bind to MAPK/ERK protein and inhibit its activity. Other inhibiting nucleic acids may bind to MAPK/ERK transcripts and inhibit the translation thereof or degrade them. Typically, such inhibiting nucleic acids may be antisense nucleic acids, morpholino oligonucleotides, inhibitory RNA molecules such as siRNAs or micro RNAs, or ribozymes. Furthermore, MAPK/ERK inhibitors may be small molecules that bind to MAPK/ERK and inhibit its activity. Such small molecule inhibitors of MAPK/ERK can be obtained by well known screening procedures or molecular modelling approaches aiming to identify compounds that bind to the active site of the MAPK/ERK kinase domain. In an aspect, the MAPK/ERK inhibitor referred to herein is a MEK inhibitor selected from the group consisting of: U0126, GSK1120212 (Trametinib), MEK162, and SCH772984. More typically, the MAPK/ERK inhibitor is GSK1120212 (Trametinib), MEK162, or SCH772984. Most typically, the MAPK/ERK inhibitor is an ERK inhibitor and, in particular, SCH772984.

The term "responder to a MAPK/ERK inhibitor" refers to a subject exhibiting a BRAF-positive cancer which upon administration of a MAPK/ERK inhibitor shows less progression, significant amelioration or cure of the cancer.

The term "sample" refers to samples comprising cancer cells or proteins and/or nucleic acids of cancer cells. Said cancer cells are derived from a single cell clone. Said samples may be derived from biopsy material from tumor tissues or body fluids as well as tissues obtained from autopsy. Body fluids can be obtained by well known techniques and include, typically, samples of blood, lymphatic fluids, alveolar, bronchial or pharyngeal lavage, liquor or urine. Tissues can be obtained by biopsy procedures which are also well known to those skilled in the art. Tissues are typically obtained from the tissue containing the tumor and comprise cancer cells or proteins and/or nucleic acids thereof.

The term "single cell clone" refers to a subpopulation and, preferably, a clonal subpopulation of cancer cells comprising a BRAF and an NRAS mutation in its genome. Single cell clones can be obtained by techniques well known to those skilled in the art. Such techniques typically include isolation of cells from body tissues or fluids, sorting of cells and growth of new cultures from each of these individual cells.

The term "BRAF", also called "v-raf murine sarcoma viral oncogene homolog B", as used herein refers to a gene encoding the BRAF protein. BRAF protein is a member of the Raf kinase family and is involved in the MAPK/ERK signaling pathway affecting cell growth and differentiation. The BRAF protein, also called B-Raf, is a serine/threonine kinase consisting of 766 amino acid in length in humans. It contains the typical Raf kinase family domains conserved region 1 (CR1), a Ras-GTP-binding self-regulatory domain, conserved region 2 (CR2), a serine-rich hinge region, and conserved region 3 (CR3), a catalytic protein kinase domain which phosphorylates a consensus sequence on protein substrates. In its active conformation, B-Raf forms dimers via hydrogen-bonding and electrostatic interactions of its kinase domains. BRAF as referred to in the context of the present invention is typically human BRAF. The protein sequence of human BRAF protein has been deposited in the NCBI database under accession number NP_004324.2, mRNA/cDNA sequences are shown under NM_004333.4 (see also SEQ ID NO: 13). A mouse BRAF protein ortholog is also known and has been deposited under NCBI database under accession number NP_647455.3, mRNA/cDNA sequences are shown under NM_139294.5. The term also encompasses variants of the aforementioned specific BRAF proteins. Such variants have at least the same essential biological and immunological properties as the specific BRAF proteins. In particular, they share the same essential biological and immunological properties if they are detectable by the same specific assays referred to in this specification, e.g., by ELISA assays using polyclonal or monoclonal antibodies specifically recognizing the said BRAF proteins. A preferred assay is described in the accompanying Examples. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific BRAF proteins. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The comparison window, preferably, is the entire length of the query sequence or at least 50% of its length. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith 1981, Add. APL. Math. 2:482, by the homology alignment algorithm of Needleman 1970, J. Mol. Biol. 48:443, by the search for similarity method of Pearson 1988, Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Moreover, the variants referred to herein include fragments of the specific BRAF proteins or the aforementioned types of variants as long as these fragments have the essential immunological and biological properties as referred to above. Such fragments may be, e.g., degradation products of the BRAF proteins. Further included are variants which differ due to posttranslational modifications such as phosphorylation. Moreover, the aforementioned BRAF proteins may be present as a monomer and/or in dimerized form.

Typical mutations in the BRAF gene of BRAF-positive cancer cells are those which cause one or more amino acid substitutions in the BRAF protein. In an aspect, said at least one mutation in the BRAF protein is a mutation resulting in an activated BRAF protein. In yet an aspect, the BRAF-positive cancer cell in accordance with the present invention has a mutated BRAF gene which encodes a BRAF protein having an amino acid substitution at a position corresponding to amino acid 600 in the human BRAF protein. It will be understood that the position of a given amino acid may vary due to amino acid deletions or additional amino acids elsewhere in the protein which occur as a result of mutagenizing events or in paralogs or orthologs of other species. Thus, a position that corresponds to, e.g., position 600 in the human BRAF protein, i.e. V600, as referred to herein also encompasses mutations in a valine which is not at position 600 due to such events provided that the said valine is flanked by the same amino acids as V600 in the human BRAF protein. The same applies mutatis mutandis to all other position numbers referred to in accordance with the present invention as positions that correspond to certain positions in a specific protein. Amino acid 600 is located in exon 15 and encoded by the base-pair 1799 in the human BRAF gene. The following amino acid substitutions have already identified at said position in human cancers: a valine-to-glutamate substitution (V600E), a valine-to-lysine substitution (V600K), a valine-to-arginine substitution (V600R), or a valine-to-aspartic acid substitution (V600D). In an aspect, the BRAF gene in BRAF-positive cells, therefore, comprises a mutation of the BRAF gene that results in an amino acid substitution at position corresponding to amino acid 600 of exon 15 of human BRAF protein. Typically, said amino acid substitution is one of the aforementioned substitutions. The BRAF gene in accordance with the present invention may have at least one mutation, i.e. may have one or more, e.g., two, three, four, five, etc., mutations including one of the aforementioned substitutions.

The term "NRAS" as used herein refers to also called "neuroblastoma RAS viral oncogene homolg", as used herein refers to a gene encoding the NRAS protein. The NRAS protein is a member of the Ras protein family and is involved as well in the MAPK/ERK signaling pathway affecting cell growth and differentiation. The NRAS protein is a GTP/GDP-binding protein having an intrinsic GTPase activity. In the GTP-bound stage, it is capable of interacting and activating Raf kinases such as the BRAF protein. The NRAS protein consists of 189 amino acid in length in humans. NRAS as referred to in the context of the present invention is typically human NRAS. The protein sequence of human NRAS protein has been deposited in the NCBI database under accession number NP_002515.1, mRNA/cDNA sequences are shown under NM_002524.4 (see also SEQ ID NO: 14). A mouse NRAS protein ortholog is also known and has been deposited under NCBI database under accession number NP_035067.2, mRNA/cDNA sequences are shown under NM_010937.2. The term also encompasses variants of the aforementioned specific NRAS proteins. Such variants have at least the same essential biological and immunological properties as the NRAS. In particular, they share the same essential biological and immunological properties if they are detectable by the same specific assays referred to in this specification, e.g., by ELISA assays using polyclonal or monoclonal antibodies specifically recognizing the said NRAS proteins. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific NRAS proteins. The degree of identity between two amino acid sequences can be determined by algorithms well known in the art and described elsewhere herein. Variants referred to above may be allelic variants or any other species specific homologs, paralogs, or orthologs. Moreover, the variants referred to herein include fragments of the specific NRAS proteins or the aforementioned types of variants as long as these fragments have the essential immunological and biological properties as referred to above. Such fragments may be, e.g., degradation products of the NRAS proteins. Further included are variants which differ due to posttranslational modifications.

In accordance with the present invention, the NRAS gene may comprise at least one mutation, i.e. one or more, e.g., two, three, four, five etc. mutations. In an aspect, said at least one mutation is a mutation resulting in the activation of the NRAS protein. In yet an aspect, the mutation of the NRAS gene results in an amino acid substitution at a position corresponding to amino acid 61 of exon 2 of the human NRAS protein. Typically, said amino acid substitution is a glutamine-to-lysine substitution (Q61K), a glutamine-to-arginine substitution (Q61R), or a glutamine-to-leucine (Q61L). Amino acid 61 is located in exon 2 and encoded by the base-pair 181 in the human NRAS gene.

Determining the presence or absence of at least one mutation in at least the NRAS gene in a sample of the subject can be carried out by various techniques on either protein or nucleic acid level.

On the protein level, the mutation can be determined based on the amino acid exchange elicited thereby. To this end, specific detection agents such as antibodies or aptamers that specifically bind to either the wild-type (i.e. non-mutated) or mutated form of the protein can be applied. If mutation specific detection agents are applied, specific binding of such agents indicates the presence of the mutation while absence of specific binding shall indicate the absence thereof.

In an aspect, the determination comprises (i) contacting the sample with a specific detection agent for a time and under conditions sufficient to allow for specific binding of the agent to the mutated NRAS protein, and (ii) detecting the specifically bound detection agent.

Specific antibodies as referred to herein, preferably, encompass to all types of antibodies which, preferably, specifically bind to NRAS. Preferably, the antibody is a monoclonal antibody, a polyclonal antibody, a single chain antibody, a chimeric antibody or any fragment or derivative of such antibodies being still capable of binding NRAS. Such fragments and derivatives comprised by the term antibody as used herein encompass a bi-specific antibody, a synthetic antibody, an Fab, F(ab)2 Fv or scFv fragment, or a chemically modified derivative of any of these antibodies. Specific binding as used in the context of the antibody of the present invention means that the antibody does not cross react with other proteins or peptides. Specific binding can be tested by various well known techniques. Antibodies or fragments thereof, in general, can be obtained by using methods which are described, e.g., in Harlow and Lane "Antibodies, A Laboratory Manual", CSH Press, Cold Spring Harbor, 1988. Monoclonal antibodies can be prepared by the techniques which comprise the fusion of mouse myeloma cells to spleen cells derived from immunized mammals and, preferably, immunized mice (Kohler 1975, Nature 256, 495, and Galfré 1981, Meth. Enzymol. 73,3). Preferably, an immunogenic peptide having the mutated portion of NRAS is applied to a mammal. The said peptide is, preferably, conjugated to a carrier protein, such as bovine serum albumin, thyroglobulin, and keyhole limpet hemocyanin (KLH). Depending on the host species, various adjuvants can be used to increase the immunological response. Such adjuvants encompass, preferably, Freund's adjuvant, mineral gels, e.g., aluminum hydroxide, and surface active substances, e.g., lysolecithin, pluronic polyols, polyanions, peptides, oil emulsions, keyhole limpet hemocyanin, and dinitrophenol. Monoclonal antibodies which specifically bind to the extracellular domain of the B-type plexin can be subsequently prepared using the well known hybridoma technique, the human B cell hybridoma technique, and the EBV hybridoma technique.

Specific aptamers as used herein are, preferably, oligonucleic acid or peptide molecules that bind to a specific target molecule (Ellington 1990, Nature 346 (6287): 818-22). Bock 1992, Nature 355 (6360): 564-6). Oligonucleic acid aptamers are engineered through repeated rounds of selection or the so called systematic evolution of ligands by exponential enrichment (SELEX technology). Peptide aptamers are designed to interfere with protein interactions inside cells. They usually comprise of a variable peptide loop attached at both ends to a protein scaffold. This double structural constraint shall increase the binding affinity of the peptide aptamer into the nanomolar range. Said variable peptide loop length is, preferably, composed of ten to twenty amino acids, and the scaffold may be any protein having improved solubility and compacity properties, such as thioredoxin-A. Peptide aptamer selection can be made using different systems including, e.g., the yeast two-hybrid system (see e.g., Hoppe-Seyler 2000,. J Mol Med. 78 (8): 426-30).

Specific antibodies and aptamers may be linked to a detectable label. Suitable detectable labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star^{™} (Amersham Biosciences), ECF^{™} (Amersham Biosciences). A suitable enzyme-substrate combination may result in a colored reaction product, fluorescence or chemiluminescence, which can be measured according to methods known in the art (e.g. using a light-sensitive film or a suitable camera system). Typical fluorescent labels include fluorescent proteins (such as GFP and its derivatives BFP, RFP and others), peptide tags, such as His-tag, FLAG-tag, Myc-tag and others, Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like.

The presence or absence of the aforementioned labels can be tested by methods and devices well known in the art including biosensors, optical devices coupled to immunoassays, analytical devices such as mass spectrometers, NMR- analyzers, or chromatography devices. Further, methods include ELISA (enzyme-linked immunosorbent assay)-based methods, fully-automated or robotic immunoassays, e.g., available on ElecsysTM analyzer, CBA which is an enzymatic Cobalt Binding Assay, available for example on Roche-HitachiTM analyzers, and latex agglutination assays, e.g., available on Roche-HitachiTM analyzers. Suitable measurement methods according the present invention also include precipitation, particularly immunoprecipitation, electrochemiluminescence, RIA (radioimmunoassay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art, such as gel electrophoresis, 2D gel electrophoresis, SDS polyacrylamid gel electrophoresis (SDS-PAGE), and Western Blotting, can be used alone or in combination with labelling or other detection methods as described above.

In yet an aspect, the mutated NRAS protein may be detected directly. To this end, differences in physical or chemical properties may be measured by mass spectroscopy or NMR based techniques. Alternatively, differences in biological activity may be measured such as increased biological activity in a cell-free or cell-based test system (activity testing).

On the nucleic acid level, the mutation can be determined by determining the nucleic acid sequence of the gene or its transcripts encoding the protein. To this end, nucleic acids or oligonucleotides that specifically bind to either the wild-type (i.e. non-mutated) or mutated form of the gene or its transcript can be applied. If mutation specific nucleic acids or oligonucleotides are applied, specific binding of such agents to the gene or its transcript or an amplicon thereof indicates the presence of the mutation while absence of specific binding shall indicate the absence thereof.

In an aspect, the determination comprises (i) contacting the sample with a specific nucleic acid or oligonucleotide for a time and under conditions sufficient to allow for specific binding of the said agent to the mutated NRAS gene or its transcript, and (ii) detecting the specifically bound nucleic acid or oligonucleotide. Typically, hybridization techniques are applied according to this aspect of the invention. Said hybridization techniques include Southern blot hybridization or Northern blot hybridization.

In yet an aspect, the determination comprises (i) contacting the sample with specific primer oligonucleotides which allow for amplification of the mutated NRAS gene only for a time and under conditions sufficient to allow for specific amplification of a portion of the said mutated NRAS gene, and (ii) detecting the amplification product. In such an aspect, the presence of an amplification product is indicative for the presence of the mutated NRAS gene, while the absence of an amplification product indicates its absence. Typically, PCR-based techniques are applied according to this aspect of the invention. Said PCR-based techniques include PCR, RT-PCR, nested PCR, qPCR, light cycle PCR, real-time PCR, irt-PCR, touchdown-PCR, multiplex-PCR, digital PCR, and others.

In a further aspect, the determination comprises performing sequencing of the mutated NRAS gene or its transcripts, in particular, of the mutated base-pair(s). Typically, conventional sequencing according to Sanger or Maxam-Gilbert may be applied. Alternatively, advanced sequencing techniques may be applied such as shotgun sequencing, bridge PCR, massively parallel signature sequencing (MPSS), polony sequencing, 454 pyrosequencing, Illumina (Solexa) sequencing, SOLiD sequencing, Ion Torrent semiconductor sequencing, DNA nanoball sequencing, heliscope single molecule sequencing, Single molecule real time (SMRT) sequencing, nanopore DNA sequencing, tunnelling currents DNA sequencing, sequencing by hybridization, sequencing with mass spectrometry, microfluidic Sanger sequencing, microscopy-based techniques, and RNAP sequencing.

More typically, the presence of the at least one mutation in exon 2 of the catalytic subunit of NRAS nucleic acid is determined by a hybridization based technology and, in particular, by
a) contacting nucleic acids in the sample from the subject with one or more of the locus-specific oligonucleotides selected from the group consisting of: GGTGAAACCTGTTTGTTGGACAT (SEQ ID NO:7); TGTATTGGTCTCTCATGGCACTGT (SEQ ID NO:8); GATAGGCAGAAATGGGCTTGA (SEQ ID NO:9); and ATCATCCTTTCAGAGAAAATAATGC (SEQ ID NO:10);
b) incubating the sample under conditions allowing specific hybridization of the oligonucleotide to its target sequence within a NRAS nucleic acid;
c) detecting said hybridization; and
d) determining the at least one mutation based on said hybridization detected in step c).

Contacting is performed such that the one or more locus-specific oligonucleotides can be in physical proximity to the nucleic acid to be detected, i.e. the nucleic acid encoding the NRAS protein having the at least one mutation (the NRAS nucleic acid).

Specific hybridization conditions which only allow hybridization of the one or more locus-specific oligonucleotides to the NRAS target sequence in the NRAS nucleic acid if the mutation is present can be determined by the person skilled in the art without further ado. The conditions may vary dependent on the locus-specific oligonucleotide(s) applied. Particular envisaged conditions are those referred to in the accompanying Examples, below.

Detection of the specific hybridization can be carried out by any technique which allows for the detection of nucleic acid hybrid of the locus-specific oligonucleotide and the target nucleic acid. Typically, the locus specific oligonucleotide may be coupled to a detectable label. Suitable detectable labels for nucleic acids in the context of hybridization techniques are well known in the art and encompass, e.g., radioactive labels, fluorescent labels, chromogenic labels, dyes, enzymatic labels, labels detectable by antibodies or aptameres, and the like. Particular envisaged labels are those referred to in the accompanying Examples, below.

Determination of the at least one mutation is carried out by detecting the specific hybridization. The information on the locus-specificity of the oligonucleotide indicates, furthermore, the kind of the mutation detected by hybridization, i.e. since the oligonucleotide has been designed to hybridize with a certain target sequence comprising, e.g., a certain mutation, the hybridization detected also indicates the presence of the said certain mutation in the target nucleic acid.

Typically, step b) further comprises the step of generating an amplification product containing the target sequence within the NRAS nucleic acid by amplifying the NRAS nucleic acid in the sample with one or both of the following oligonucleotide primers: forward oligonucleotide primer having SEQ ID NO:11 and reverse oligonucleotide primer having SEQ ID NO: 12.

The amplification can be carried out by PCR as specified elsewhere herein in detail, i.e. the reverse and forward primers are allowed to anneal to the target sequence such that DNA synthesis can occur. Subsequently, the newly synthesized DNA strands are dissociated and the cycle is started again. Typically, the amplification PCR is carried out for 15 to 45 cycles, more typically for 16 to 40 cycles and even more typically for 16 to 30 cycles. Suitable PCR conditions depend on the applied forward and reverse primers and can be determined by those skilled in the art without further ado. Particular PCR conditions envisaged in accordance with the present invention are those specified in the accompanying Examples, below.

In order to further strengthen the assessment made by the method of the present invention, it is also envisaged that in addition to NRAS, other cancer biomarkers as well. In an aspect, the method further encompasses determining the presence or absence of at least one mutation in the BRAF gene, whereby the presence of the said at least one mutation further identifies the subject as a non-responder to a BRAF inhibitor and a responder to a MAPK/ERK inhibitor. The at least one BRAF mutation to be determined is, typically, one of the BRAF amino acid substitutions referred to before. The said BRAF mutation can be determined on the protein or nucleic acid level as well in a manner analogous to the determination of the at least one NRAS mutation specified elsewhere herein.

More typically, the presence of the at least one mutation in exon 15 of the catalytic subunit of BRAF nucleic acid is determined by a hybridization based technology and, in particular, by
a) contacting nucleic acids in the sample from the subject with one or more of the locus-specific oligonucleotides selected from the group consisting of: CTAAGAGGAAAGATGAAGTACTATG (SEQ ID NO:1); CTAGTAACTCAGCAGCATCTCAG (SEQ ID NO:2); CTACTGTTTTCCTTTACTTACTACACCTCAGA (SEQ ID NO:3); and ATCCAGACAACTGTTCAAACTGAT(SEQ ID NO:4);
b) incubating the sample under conditions allowing specific hybridization of the oligonucleotide to its target sequence within a BRAF nucleic acid;
c) detecting said hybridization; and
d) determining the at least one mutation based on said hybridization detected in step c).

Contacting is performed such that the one or more locus-specific oligonucleotides can be in physical proximity to the nucleic acid to be detected, i.e. the nucleic acid encoding the BRAF protein having the at least one mutation (the BRAF nucleic acid).

Specific hybridization conditions which only allow hybridization of the one or more locus-specific oligonucleotides to the BRAF target sequence in the BRAF nucleic acid if the mutation is present can be determined by the person skilled in the art without further ado. The conditions may vary dependent on the locus-specific oligonucleotide(s) applied. Particular envisaged conditions are those referred to in the accompanying Examples, below.

Detection of the specific hybridization can be carried out by any technique which allows for the detection of nucleic acid hybrid of the locus-specific oligonucleotide and the target nucleic acid. Typically, the locus specific oligonucleotide may be coupled to a detectable label. Particular envisaged labels are those referred to in the accompanying Examples, below.

Determination of the at least one mutation is carried out by detecting the specific hybridization. The information on the locus-specificity of the oligonucleotide indicates, furthermore, the kind of the mutation detected by hybridization, i.e. since the oligonucleotide has been designed to hybridize with a certain target sequence comprising, e.g., a certain mutation, the hybridization detected also indicates the presence of the said certain mutation in the target nucleic acid.

Typically, step b) further comprises the step of generating an amplification product containing the target sequence within the BRAF nucleic acid by amplifying the NRAS nucleic acid in the sample with one or both of the following oligonucleotide primers: forward oligonucleotide primer having SEQ ID NO:5 and reverse oligonucleotide primer having SEQ ID 6.

The amplification can be carried out by PCR as specified elsewhere herein. Particular PCR conditions envisaged in accordance with the present invention are those specified in the accompanying Examples, below.

If the at least one mutation in the NRAS gene has been determined as set forth above, the subject is to be identified as a non-responder to a BRAF inhibitor and a responder to a MAPK/ERK inhibitor. Usually, the said identification will lead to a recommendation of therapeutic measures to be applied to the said subject. As discussed elsewhere herein, it has been found that a subject having at least one mutation in the NRAS protein in accordance with the invention will be a non-responder to BRAF inhibitors but, at the same time, will respond to MAPK/ERK inhibitors. Accordingly, it is envisaged in accordance with the present invention that a recommendation of a suitable therapy can be given to such a subject upon proper identification. Therefore, in an aspect, the method of the invention further comprises recommending to the subject the administration of a MAPK/ERK inhibitor, in particular, a MAPK/ERK inhibitor as specified herein, if the subject has been identified as a non-responder to a BRAF inhibitor and a responder to a MAPK/ERK inhibitor. In yet an aspect, the method may further comprise administering to the subject said MAPK/ERK inhibitor, in particular, a MAPK/ERK inhibitor as specified herein, and, in still an aspect, adjusting the dosage of or refraining from the administration of a BRAF inhibitor, in particular, a BRAF inhibitor as specified herein.

To better characterize the evolution of intra-patient heterogeneity under different treatment regimens, in the studies underlying the present invention, exome sequencing on multiple samples from three stage IV melanoma patients who each received a different therapy but progressed quickly under treatment was performed. Surplus biopsy material from different stages (depending on availability) was used including blood, dysplastic nevi, primary tumors and, metastases before treatment as well as metastases after death obtained during autopsy. To better characterize intra-tumor heterogeneity, multiple histologically distinct regions were sequenced of the same primary tumor when possible and single-cell clones were made from early passage cultures for targeted re-sequencing. The confluence of increasingly more specific targeted pathway inhibitor pipelines and the application of powerful next-generation sequencing technologies have, advantageously, allowed for an improved characterization and treatment approach tailored to the key driver pathways most relevant to metastatic melanoma progression ^{2,22,23}.

Specifically, in order to better characterize how individual cancer patients respond to standard therapies, three patients with similar treatment time courses, but different oncogenic mutations and therapeutic regimens have been identified. The first patient had a BRAFV600E mutation and had an initial response to targeted BRAF-inhibitor therapy. Patient 2 was homozygous wild-type for both BRAF and NRAS, and received pazopanib, which is a multi-receptor tyrosine kinase inhibitor. Lastly, patient 3 had an NRASQ61R mutation, and was administered a MEK-inhibitor. Whole exome sequencing data were generated from punches of FFPE material obtained from multiple biopsies and were referenced to germline DNA isolated from each patient's blood. This approach provided a more comprehensive view of intra-patient genomic heterogeneity than earlier studies that investigated larger patient cohorts, but with fewer samples from each patient.

By analyzing high-quality single nucleotide variations (SNVs) present in the patient tumors, it could be show that each patient's primary tumors contained the largest genetic diversity compared to all of their metastases. This is consistent with the expectation that the site of cancer origin would contain more genetic variants than the descendants that arose later and presumably had less time for the acquisition of de novo mutations. Interestingly, both dysplastic nevi from patient 1 had a lower protein-coding mutational burden than any of the tumor samples sequenced from the three patients. Although the reason for this is unclear, the reduced genetic diversity of the nevi may be the result of less genomic instability or possibly a shorter time period to accumulate mutations, amongst other possible causes.

Whole-exome phylogenetic analysis of these data was further used to infer the evolutionary relationships between the tumors within each patient, and to determine how each therapeutic regimen affected the evolution of genetic heterogeneity. Unlike in previous studies that showed a branching evolution of clones subsequent to targeted therapy, it could be seen that a strong, well-supported monophyletic evolution of metastases following both BRAF and MEK inhibitor treatment arises and relapses. In contrast, patient 2, who received a multi-kinase inhibitor (i.e. pazopanib), did not have a monophyletic topology of late tumor metastases, which is suggestive of genetic drift between the late metastases.

Interestingly, despite the monophyletic segregation of late metastases in the patient who received the BRAF inhibitor, no known mechanism of resistance was shared between all sequenced biopsies. In fact, the activating mutation NRASQ61K was identified by both Sanger sequencing and digital PCR to be present in a single metastasis of patient 1, but absent in all other resistant tumor samples from that patient. This is consistent with previously published data showing heterogeneity in resistance mechanisms within individual patients ¹¹, and exacerbates the efforts to both catalog the causes and treat patients who have developed therapeutic resistance. Thus, the different metastases likely contain divergent mechanisms of resistance, although we observed a monophyletic selection of subclones subsequent to treatment.

By isolating and sequencing colonies derived from 26 single cell clones of this resistant tumor, it could be shown for the first time that both activating MAPK mutations were present in a single tumor cell. These double-mutated cells grew in normal culturing conditions, were resistant to the BRAF-inhibitor with which the patient had been treated, but were only partially resistant to two other BRAF-inhibitors. A reduction in pERK levels could still be observed in the presence of LGX818 and PLX4032, although the cells remained resistant to BRAF inhibition. Importantly, the double-mutated cells remained sensitive to combined MEK and BRAF inhibition, as well as mono-agent MEK and ERK inhibition. This observation suggests that simultaneous or second-line treatment with other MAPK-pathway inhibitors and, in particular, MAPK/ERK inhibitors, may still be effective in controlling progression, despite the presence of resistance-conferring mutations.

However, as the double-mutated genotype was only present in late metastasis #6 out of the other 5 metastases of patient 1 and the underlying mechanisms that conferred therapeutic resistance on the other tumors remain unclear, the efficacy of these second-line or combination treatments in controlling overall tumor burden is questionable. This would be especially true if the other tumors in patient 1 activated different pathways, such as PI3K, PTEN, and AKT, thereby rendering them insensitive to MAPK inhibition. By digital PCR, it was demonstrated that the frequency of double-mutated cells is variable even within a single resistant tumor, suggesting that these cells may also contribute to resistance in a paracrine manner or may have intra-tumor heterogeneity in resistance mechanisms.

The demonstration of monophyletic evolution of cancer cells in patients who received targeted inhibition in the studies underlying the present invention suggests a selection of heterogeneous subclones that could better survive that therapeutic environment. However, the apparent lack of a common mechanism of resistance between these tumors indicates that the subsequent emergence of resistance may have occurred through a shared genetic mechanism not identifiable by our approaches, through non-genetic means, or in a divergent way in each individual metastasis. All of those possibilities pose serious therapeutic challenges. But the remaining sensitivity to MAPK-inhibition of the double-mutated melanoma cells suggests that combination and second-line therapies using MAPK-pathway inhibitors instead or in addition to, e.g., BRAF inhibitors in the context of precision medicine may still be effective if they consider the spatial and temporal genetic heterogeneity present in metastatic melanoma patients.

Thanks to the present invention, it is now possible to characterize cancer and, in particular, cancer with BRAF-positive cancer cells for resistance to BRAF inhibitors and to select more effective therapies for those patients that are resistant. Moreover, the present invention also provides for more efficient therapies based on the use of MEK/ERK inhibitors in patients which suffer from BRAF-positive cancers that exhibit resistance to BRAF inhibitors. In general, the studies underlying the present invention have also provided for a diagnostic method for diagnosing or assessing cancer, in particular, with respect to double-mutant cancer cells carrying at least one NRAS and at least one BRAF mutation.

The definitions explanations of the terms made herein above apply mutatis mutandis for the following embodiments.

In the following, typical embodiments of the present invention are described:
In an embodiment of the method of the invention, said method further comprises determining the presence or absence of at least one mutation in the BRAF gene, whereby the presence of the said at least one mutation further identifies the subject as a non-responder to a BRAF inhibitor and a responder to a MAPK/ERK inhibitor.
In another embodiment of the method of the invention, the BRAF-positive cancer is melanoma cancer.

In a further embodiment of the method of the present invention, the BRAF-positive cancer is comprised of a cell population derived from a single cell clone.

In yet an embodiment of the method of the present invention, the cells of the cell population contain in their genome at least one mutation in the BRAF gene and at least one mutation in the NRAS gene.

In yet an embodiment of the method of the invention, the BRAF-inhibitor is a small molecule inhibitor of BRAF activity. Typically, said small molecule inhibitor of BRAF activity is LGX818, PLX4032 and/or GSK2118436.

In an embodiment of the method of the invention, the said MAPK/ERK inhibitor is a small molecule inhibitor of MEK or ERK activity. Typically, said inhibitor of MEK activity is GSK1120212 or MEK162, and said inhibitor of ERK activity is SCH772984.

In yet an embodiment of the method of the invention, the mutation of the NRAS gene results in an amino acid substitution at a position corresponding to amino acid 61 of exon 2 of the human NRAS protein. Typically, said amino acid substitution is a glutamine-to-lysine substitution (Q61K), a glutamine-to-arginine substitution (Q61R), or a glutamine-to-leucine (Q61L).

In a further embodiment of the method of the invention, the mutation of the BRAF gene results in an amino acid substitution at position corresponding to amino acid 600 of exon 15 of human BRAF protein. Typically, said amino acid substitution is a valine-to-glutamate substitution (V600E), a valine-to-lysine substitution (V600K), a valine-to-arginine substitution (V600R), or a valine-to-aspartic acid substitution (V600D).

In yet an embodiment of the method of the invention, said sample comprises a BRAF-positive cancer cell.

In a further embodiment of the method of the invention, said sample is selected from the group consisting of tissue resection samples, tissue biopsy samples, primary tumor samples, samples of metastatic lesion, or samples comprising circulating tumor cells including blood..

In an embodiment of the method of the present invention, the presence of the at least one mutation in exon 2 of the catalytic subunit of NRAS nucleic acid is determined by
a) contacting nucleic acids in the sample from the subject with one or more of the locus-specific oligonucleotides selected from the group consisting of: GGTGAAACCTGTTTGTTGGACAT (SEQ ID NO:7); TGTATTGGTCTCTCATGGCACTGT (SEQ ID NO:8); GATAGGCAGAAATGGGCTTGA (SEQ ID NO:9); and ATCATCCTTTCAGAGAAAATAATGC (SEQ ID NO:10);
b) incubating the sample under conditions allowing specific hybridization of the oligonucleotide to its target sequence within a NRAS nucleic acid;
c) detecting said hybridization; and
d) determining the at least one mutation based on said hybridization detected in step c).

Typically, step b) further comprises the step of generating an amplification product containing the target sequence within the NRAS nucleic acid by amplifying the NRAS nucleic acid in the sample with one or both of the following oligonucleotide primers: forward oligonucleotide primer having SEQ ID NO:11 and reverse oligonucleotide primer having SEQ ID NO: 12.

In an embodiment of the method of the present invention, the presence of the at least one mutation in exon 15 of the catalytic subunit of BRAF nucleic acid is determined by
a) contacting nucleic acids in the sample from the subject with one or more of the locus-specific oligonucleotides selected from the group consisting of: CTAAGAGGAAAGATGAAGTACTATG (SEQ ID NO:1); CTAGTAACTCAGCAGCATCTCAG (SEQ ID NO:2); CTACTGTTTTCCTTTACTTACTACACCTCAGA (SEQ ID NO:3); and ATCCAGACAACTGTTCAAACTGAT(SEQ ID NO:4);
b) incubating the sample under conditions allowing specific hybridization of the oligonucleotide to its target sequence within a BRAF nucleic acid;
c) detecting said hybridization; and
d) determining the at least one mutation based on said hybridization detected in step c).

Typically, step b) further comprises the step of generating an amplification product containing the target sequence within the BRAF nucleic acid by amplifying the NRAS nucleic acid in the sample with one or both of the following oligonucleotide primers: forward oligonucleotide primer having SEQ ID NO:5 and reverse oligonucleotide primer having SEQ ID 6.

In yet an embodiment of the method of the invention, said method further comprises recommending to the subject the administration of a MAPK/ERK inhibitor drug if the subject has been identified as a non-responder to a BRAF inhibitor and a responder to a MAPK/ERK inhibitor.

The present invention also relates to an MAPK/ERK inhibitor for use in treating a subject suffering from a BRAFpositive cancer, whereby the said cancer has been found to comprise cancer cells carrying (i) at least one mutation in the NRAS gene or (ii) at least one mutation in the NRAS gene and at least one mutation in the BRAF gene. In addition, the use of an MAPK/ERK inhibitor for the preparation of a medicament for the treatment of a BRAF-positive cancer patient, whereby the said cancer has been found to (i) at least have at least one mutation in the NRAS gene or (ii) at least have at least one mutation in the NRAS gene and at least one mutation in the BRAF gene is contemplated according to the invention.

Thus, the MAPK/ERK inhibitor shall be used for treating as medicament and may be accordingly formulated as such. The term "medicament" as used herein refers, in one aspect, to a pharmaceutical composition containing the inhibitor referred to above as pharmaceutical active compound, wherein the pharmaceutical composition may be used for human or non-human therapy of the diseases specified herein in a therapeutically effective dose. The inhibitor, typically, can be present in liquid or lyophilized form. The medicament is, in an aspect, for topical or systemic administration. Conventionally, a medicament will be administered intramuscular or, subcutaneous. However, depending on the nature and the mode of action of a compound, the medicament may be administered by other routes as well. The inhibitor shall be the active ingredient of the composition, and is, typically, administered in conventional dosage forms prepared by combining the drug with standard pharmaceutical carriers according to conventional procedures. These procedures may involve mixing, granulating, and compression, or dissolving the ingredients as appropriate to the desired preparation. It will be appreciated that the form and character of the pharmaceutical acceptable carrier or diluent is dictated by the amount of active ingredient with which it is to be combined, the route of administration, and other well-known variables. A carrier must be acceptable in the sense of being compatible with the other ingredients of the formulation and being not deleterious to the recipient thereof. The pharmaceutical carrier employed may include a solid, a gel, or a liquid. Examples for solid carriers are lactose, terra alba, sucrose, talc, gelatin, agar, pectin, acacia, magnesium stearate, stearic acid and the like. Exemplary of liquid carriers are phosphate buffered saline solution, syrup, oil, water, emulsions, various types of wetting agents, and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania. A diluent is selected so as not to affect the biological activity of the combination. Examples of such diluents are distilled water, physiological saline, Ringer's solutions, dextrose solution, and Hank's solution. In addition, the pharmaceutical composition or formulation may also include other carriers, adjuvants, or nontoxic, non-therapeutic, non-immunogenic stabilizers and the like. A therapeutically effective dose refers to an amount of the compound to be used in medicament according to the present invention which prevents, ameliorates or treats the symptoms accompanying a disease referred to in this specification. Therapeutic efficacy and toxicity of the compound can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. The dosage regimen will be determined by the attending physician and other clinical factors. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment. The medicament referred to herein is administered at least once in order to treat or ameliorate or prevent a disease or condition recited in this specification. However, the said medicament may be administered more than one time. Specific medicaments are prepared in a manner well known in the pharmaceutical art and comprise at least one active compound referred to herein above in admixture or otherwise associated with a pharmaceutically acceptable carrier or diluent. For making those specific pharmaceutical compositions, the active compound(s) will usually be mixed with a carrier or the diluent. The resulting formulations are to be adapted to the mode of administration. Dosage recommendations shall be indicated in the prescribers or users instructions in order to anticipate dose adjustments depending on the considered recipient. The medicament according to the present invention may, in a further aspect, of the invention comprise drugs in addition to the MAPK/ERK inhibitor which are added to the medicament during its formulation. Details on such drugs are to be found elsewhere herein. Finally, it is to be understood that the formulation of a medicament takes place under GMP standardized conditions or the like in order to ensure quality, pharmaceutical security, and effectiveness of the medicament.

It follows from the above that the MAPK/ERK inhibitor may also be used in a method of treating BRAF-positive cancer in a subject suffering therefrom, said method comprises administering to the subject a therapeutically effective amount of a MAPK/ERK inhibitor.

Disclosed is a method for diagnosing cancer in a sample of a subject suspected to suffer from cancer comprising: a) generating one or more amplification products containing target sequences within the BRAF nucleic acid and the NRAS nucleic acid by amplifying nucleic acids in the sample with two of the following primer oligonucleotides:
CTAAGAGGAAAGATGAAGTACTATG (SEQ ID NO:1); CTAGTAACTCAGCAGCATCTCAG (SEQ ID NO:2); CTACTGTTTTCCTTTACTTACTACACCTCAGA (SEQ ID NO:3); and/or ATCCAGACAACTGTTCAAACTGAT (SEQ ID NO:4) and with two of the following primer oligonucleotides: GGTGAAACCTGTTTGTTGGACAT (SEQ ID NO:7); TGTATTGGTCTCTCATGGCACTGT (SEQ ID NO:8); GATAGGCAGAAATGGGCTTGA (SEQ ID NO:9); and/or ATCATCCTTTCAGAGAAAATAATGC (SEQ ID NO:10);
b) contacting the nucleic acid sample with one or more of the following mutation-specific BRAF oligonucleotides: CTAAGAGGAAAGATGAAGTACTATG (SEQ ID NO:1); CTAGTAACTCAGCAGCATCTCAG (SEQ ID NO:2); CTACTGTTTTCCTTTACTTACTACACCTCAGA (SEQ ID NO:3); and/or ATCCAGACAACTGTTCAAACTGAT (SEQ ID NO:4); and with one or more of the following location-specific NRAS oligonucleotides: GGTGAAACCTGTTTGTTGGACAT (SEQ ID NO:7); TGTATTGGTCTCTCATGGCACTGT (SEQ ID NO:8); GATAGGCAGAAATGGGCTTGA (SEQ ID NO:9); and/or ATCATCCTTTCAGAGAAAATAATGC (SEQ ID NO:10);
c) incubating the sample under conditions allowing specific hybridization of the oligonucleotides to their respective target sequences within the BRAF nucleic acid and the NRAS nucleic acid;
d) detecting said hybridization, whereby cancer is diagnosed.

The term "diagnosing" as used herein means assessing whether a subject as referred to herein suffers from cancer (i.e. rule-in into the cancer group of patients), or not (i.e. rule-out). As will be understood by those skilled in the art, such an assessment is usually not intended to be correct for 100% of the subjects to be diagnosed. The term, however, requires that assessment of the presence or absence of cancer is correct for a statistically significant portion of the subjects (e.g. a cohort in a cohort study). Whether a portion is statistically significant can be determined as described elsewhere herein.

The term "cancer" as used herein refers to all malignant neoplasms characterized by abnormal cell growth and invasiveness. In particular, the cancer referred to herein is a BRAF-positive cancer as specified elsewhere herein.

The phrase "generating one or more amplification products" as referred herein can be achieved by any primer-based nucleic acid amplification technique. In an aspect, the generation is achieved by PCR-based techniques referred to in detail elsewhere herein or n the accompanying Examples.

In an embodiment of the aforementioned method, said cancer is derived from a single cell clone.

The invention also encompasses a kit for diagnosing cancer, typically, derived from a single cell clone, in a sample of a subject comprising the following oligonucleotides: CTAAGAGGAAAGATGAAGTACTATG (SEQ ID NO:1); CTAGTAACTCAGCAGCATCTCAG (SEQ ID NO:2); CTACTGTTTTCCTTTACTTACTACACCTCAGA (SEQ ID NO:3); ATCCAGACAACTGTTCAAACTGAT (SEQ ID NO:4); GGTGAAACCTGTTTGTTGGACAT (SEQ ID NO:7); TGTATTGGTCTCTCATGGCACTGT (SEQ ID NO:8); GATAGGCAGAAATGGGCTTGA (SEQ ID NO:9); and ATCATCCTTTCAGAGAAAATAATGC (SEQ ID NO:10).

The term "kit" as used herein refers to a collection of the aforementioned components, typically, provided in separately or within a single container. The container also comprises instructions for carrying out the method of the present invention. These instructions may be in the form of a manual or may be provided by a computer program code which is capable of carrying out the identification referred to in the methods of the present invention and to establish a diagnosis accordingly when implemented on a computer or a data processing device. The computer program code may be provided on a data storage medium or device such as an optical storage medium (e.g., a Compact Disc) or directly on a computer or data processing device. Further, the kit may comprise positive and negative control target nucleic acids. The kit, in an aspect may also comprise other components required for performing the method of the invention, such as detection agents, e.g., an antibody, buffers, other reagents required for detection, for example, conjugate and/or substrates and the like.

Further encompassed by the invention is a device for diagnosing cancer, typically, derived from a single cell clone, in a sample of a subject suspected to suffer from cancer and/or for identifying whether a subject suffering from a BRAF-positive cancer is a non-responder to a BRAF inhibitor, or not, and/or is a responder to an MAPK/ERK inhibitor comprising:
(i) an analyzing unit comprising one or more of the following mutation-specific BRAF oligonucleotides: CTAAGAGGAAAGATGAAGTACTATG (SEQ ID NO:1); CTAGTAACTCAGCAGCATCTCAG (SEQ ID NO:2); CTACTGTTTTCCTTTACTTACTACACCTCAGA (SEQ ID NO:3); and/or ATCCAGACAACTGTTCAAACTGAT (SEQ ID NO:4) and one or more of the following location-specific NRAS oligonucleotides: GGTGAAACCTGTTTGTTGGACAT (SEQ ID NO:7); TGTATTGGTCTCTCATGGCACTGT (SEQ ID NO:8); GATAGGCAGAAATGGGCTTGA (SEQ ID NO:9); and/or ATCATCCTTTCAGAGAAAATAATGC (SEQ ID NO:10) and
(ii) a detector which is capable detecting specific hybridization of BRAF and NRAS nucleic acids to said oligonucleotides.

The term "device" as used herein relates to a system comprising the aforementioned components operatively linked to each other as to allow the diagnosis or identification according to the methods of the invention. The analysing unit, in an aspect, comprises said oligonucleotides in immobilized form on a solid support which is to be contacted to the sample comprising the target nucleic acids to be determined. The analysing unit may further comprise or be operatively linked to vials comprising washing and hybridization solutions for carrying out the hybridization reaction.

The detector is adapted to detect the specific hybridization of the oligonucleotides and the target nucleic acids. Dependent on the label used for the oligonucleotides, different detectors may be used, e.g., optical detectors may be applied in the case of fluorescent labels or dyes.

The device may further comprise a computing device for data evaluation. A computing device may be a general purpose computer or a portable computing device, for example. It should also be understood that multiple computing devices may be used together, such as over a network or other methods of transferring data, for performing one or more steps of the methods disclosed herein. Exemplary computing devices include desktop computers, laptop computers, personal data assistants and smart phones, cellular devices, tablet computers, servers, and the like. In general, a computing device comprises a processor capable of executing a plurality of instructions (such as a program of software). A computing device has access to a memory. A memory is a computer readable medium and may comprise a single storage device or multiple storage devices, located either locally with the computing device or accessible to the computing device across a network, for example. Computer-readable media may be any available media that can be accessed by the computing device and includes both volatile and non-volatile media. Further, computer readable-media may be one or both of removable and non-removable media. By way of example, and not limitation, computer-readable media may comprise computer storage media. Exemplary computer storage media includes, but is not limited to, RAM, ROM, EEPROM, flash memory or any other memory technology, CD-ROM, Digital Versatile Disk (DVD) or other optical disk storage, magnetic cassettes, magnetic tape, magnetic disk storage or other magnetic storage devices, or any other medium which can be used for storing a plurality of instructions capable of being accessed by the computing device and executed by the processor of the computing device.

The computing device may also have access to an output device. Exemplary output devices include fax machines, displays, printers, and files, for example. According to some embodiments of the present disclosure, a computing device may perform one or more steps of a method disclosed herein, and thereafter provide an output, via an output device, relating to a result of the method.

The invention envisages a method of assessing responsiveness to targeted therapy against cancer, typically, derived from a single cell clone, in a patient comprising:
a) providing a sample from the patient,
b) testing the sample for the presence of mutations in the BRAF and the NRAS genes, wherein testing is performed by one of the methods selected from a group consisting of selective amplification, probe hybridization or nucleic acid sequencing;
c) if mutations in the NRAS and BRAF genes are detected, detecting responsiveness to a MAPK/ERK inhibitor and non-responsiveness to a BRAF inhibitor.

In an aspect of the aforementioned method, the presence of mutations in the BRAF and NRAS genes are determined selective amplification, probe hybridization or nucleic acid sequencing as described elsewhere herein in detail. In particular, the locus-specific or mutation specific oligonucleotides or the primer oligonucleotides specified elsewhere herein may be used.

Furthermore, the invention relates to a method of assessing cancer, typically, derived from a single cell clone, in a patient comprising:
a) providing or obtaining a sample from the patient containing nucleic acids,
b) contacting the nucleic acids in the sample with a nucleic acid probe specific for mutations in the BRAF and NRAS genes,
c) if mutations in the NRAS and BRAF genes are detected, assessing the cancer as responsive to a MAPK/ERK inhibitor and non-responsive to a BRAF inhibitor.

Yet further, the invention relates to a method of assessing cancer, typically, derived from a single cell clone, in a patient comprising:
a) providing or obtaining a sample from the patient containing nucleic acids,
b) contacting the nucleic acids in the sample with a nucleic acid probe specific for mutations in the BRAF and NRAS genes,
c) if mutations in the NRAS and BRAF genes are detected, reporting that the cancer is responsive to a MAPK/ERK inhibitor and non-responsive to a BRAF inhibitor.

In an aspect of the aforementioned method, nucleic acid probe specific for mutations in the BRAF and NRAS genes are the locus-specific or mutation specific oligonucleotides specified elsewhere herein.

All references referred to throughout this specification are herewith incorporated by reference with respect to their disclosure content specifically mentioned and its entirety.

### FIGURES

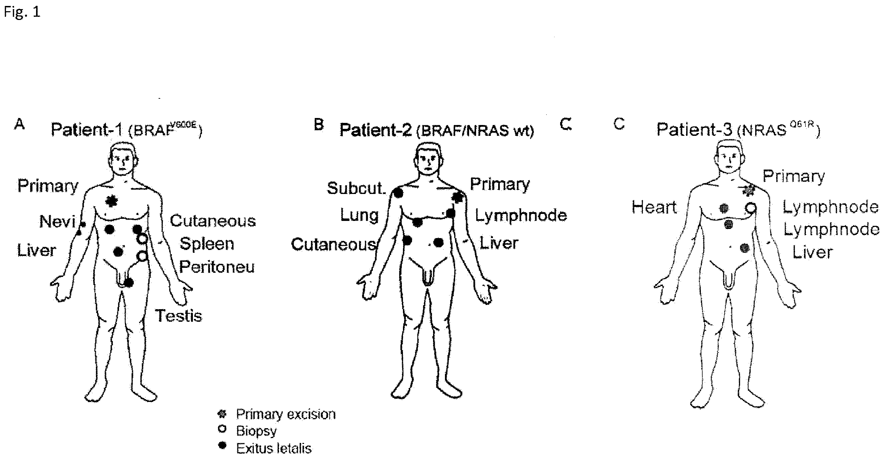

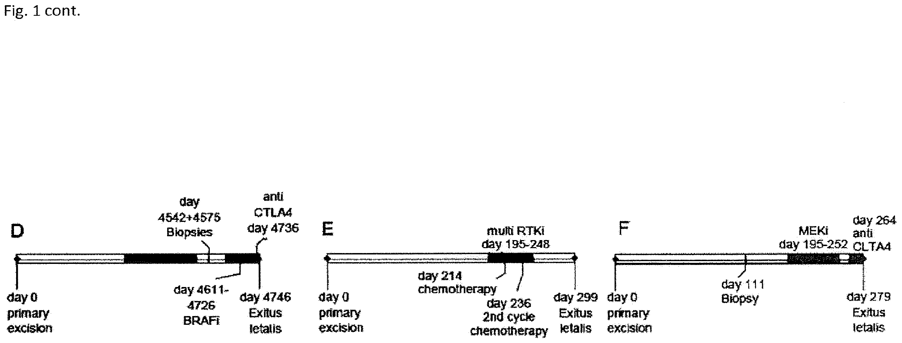

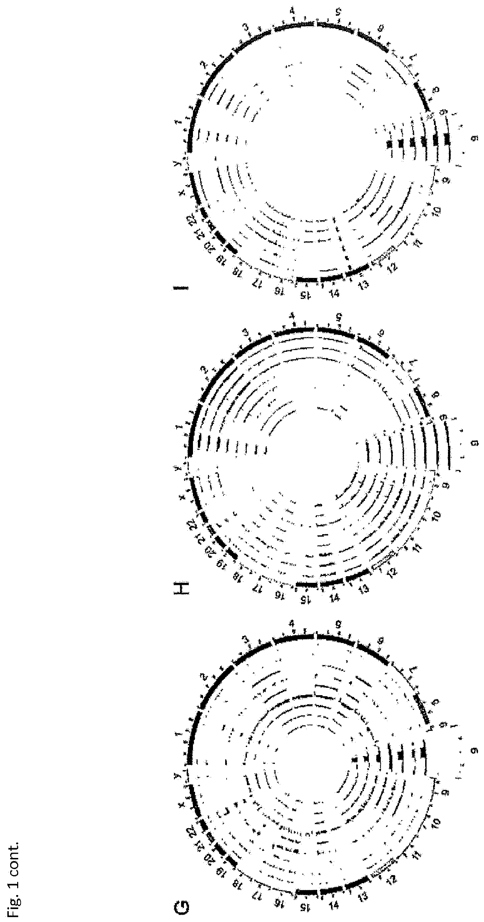

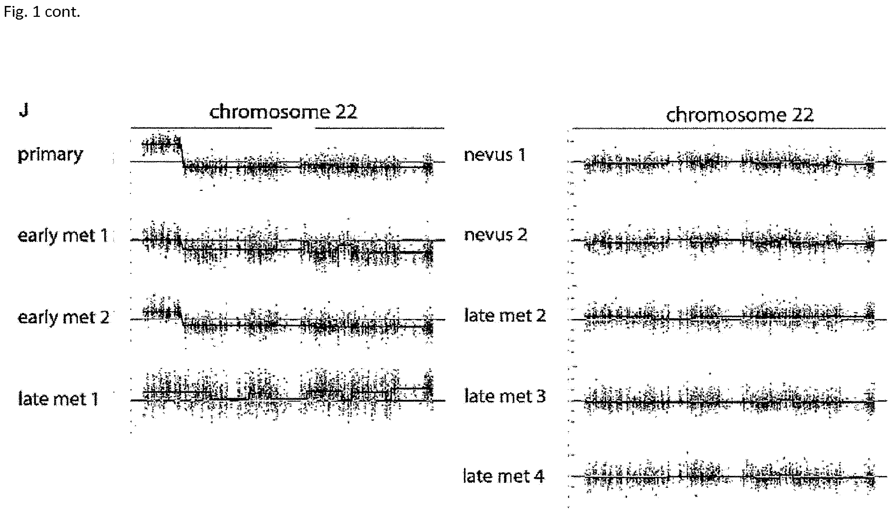

**Fig.1**: Patient cohort and copy number variations. (A) Samples from patient 1 included the primary tumor, two dysplastic nevi, two early metastases and 4 late metastases after tumor relapse. (B) Patient 1 had a BRAFV600E mutated melanoma and received first IFNa treatment followed by a specific BRAF inhibitor treatment to which he responded but then became resistant. (C) Patient 2 was diagnosed with a melanoma that was wildtype for both BRAF and NRAS. The primary tumor was punched and sequenced three times. Additionally five late metastases were sequenced. (D) Patient received the multi receptor tyrosine kinase inhibitor (Pazopanib), to which he responded but then became resistant. (E) Patient 3 had an NRASQ61R mutation. The primary tumor was punched two times and biopsies were taken from one early and three late metastases. (F) Patient received the MEK inhibitor GSK1120212, to which he responded but then became resistant followed by a short period of anti-CTLA4 treatment. (G) The copy number variations (CNVs) are plotted using Circos. Every ring shows the CNVs detected by Excavator of one biopsy, starting with two nevi in the two outermost circles followed by the primary tumor, the two early metastases and finally the late metastases 1 to 4. (H) displays the CNVs of patient 2 in from outside to the center: primary tumor samples 1 to 3 and the late metastases 1 to 5. (I) shows the same for patient 3, from outside towards the center: the primary tumor samples 1 and 2, one early metastases and the late metastases 1 to 3. The enlarged regions show a commonly lost region in chromosome 9 which is coding for the tumor suppressor CDKN2A. (K) Copy number variations in chromosome 22 of patient 1 show high degree of heterogeneity. The primary tumor has a gain in a region of 22p and a loss in a large area of 22p and 22q. The gain, but not the loss can be seen in the early met 1 but in no other metastasis. The loss, but not the gain, can be found in the early met 2 and late metastasis 1 but no other metastasis.

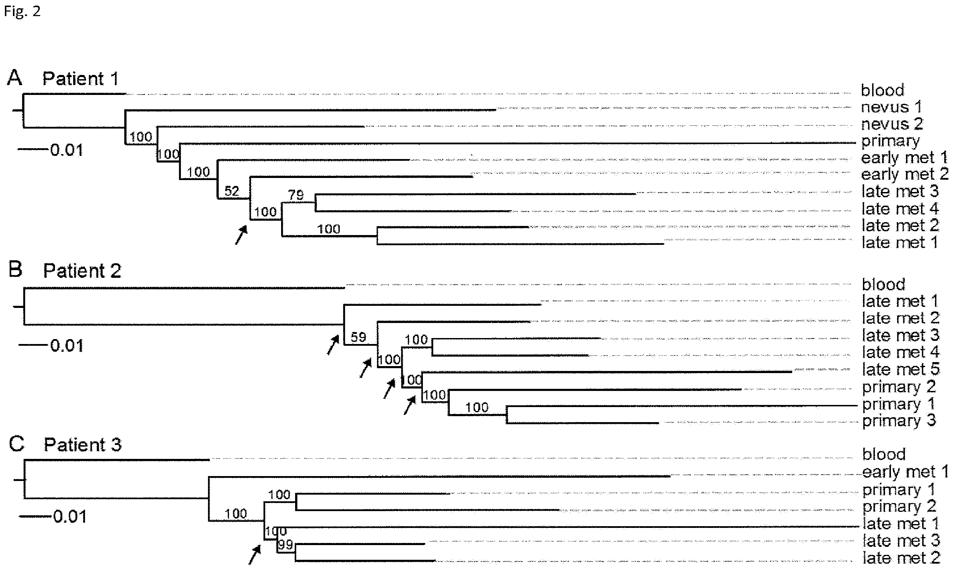

**Fig.2****:** Whole-exome phylogenetic trees of patient biopsies. Branch-lengths represent relative distances based on SNVs and indels, and the branches are colored according to biopsy type. Maximum likelihood phylogenetic trees are rooted by the blood sample for patient 1 (A), patient 2 (B), and patient 3 (C). Node supports are given as bootstrap values, with greater than 50% considered to be strong support.

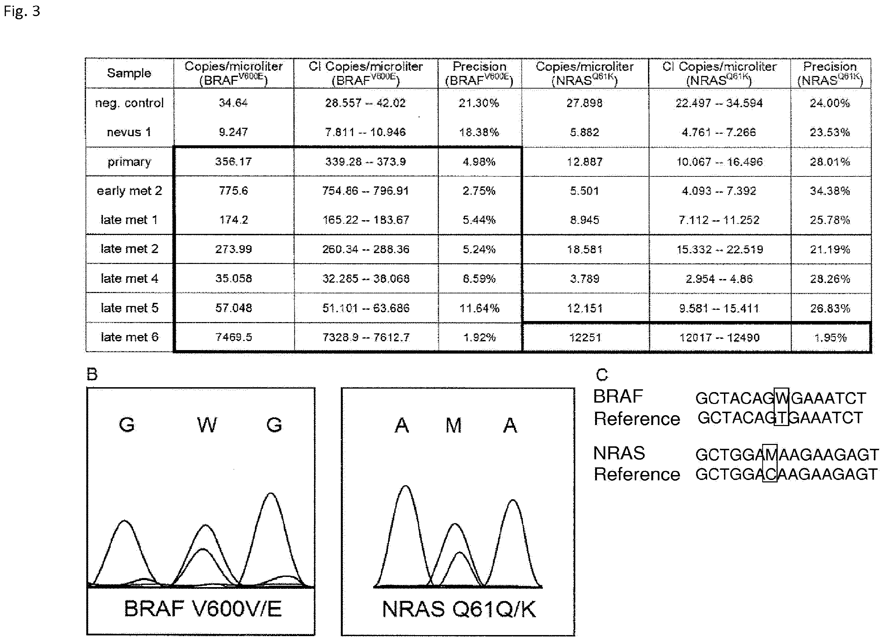

**Fig.3****:** Digital PCR and Sanger sequencing of patient 1 samples. (A) dPCR using a probe against BRAFV600E and NRASQ61K showed BRAFV600E mutated DNA in all tumor samples. dPCR reactions positive for NRASQ61K could be detected only in the late metastasis 6 of this patient. Precision values of less than 15% are considered to be highly reproducible, positive reactions. (B) representative spectrogram and (C) sequences from Sanger sequencing of 26 cell cultures grown from single melanoma cells isolated from late metastasis 6. All 26 clonal cultures had both the BRAFV600E and NRASQ61K mutations.

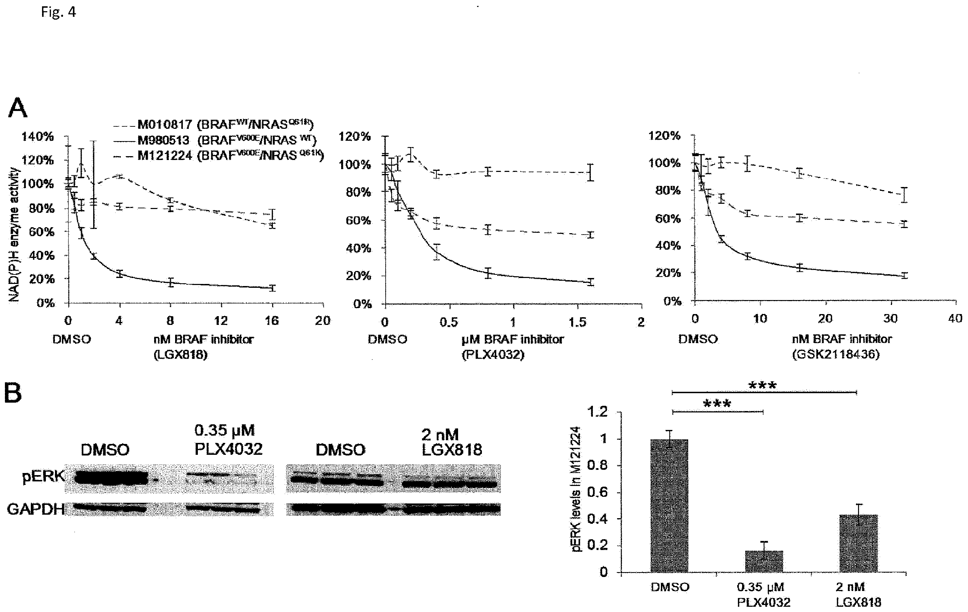

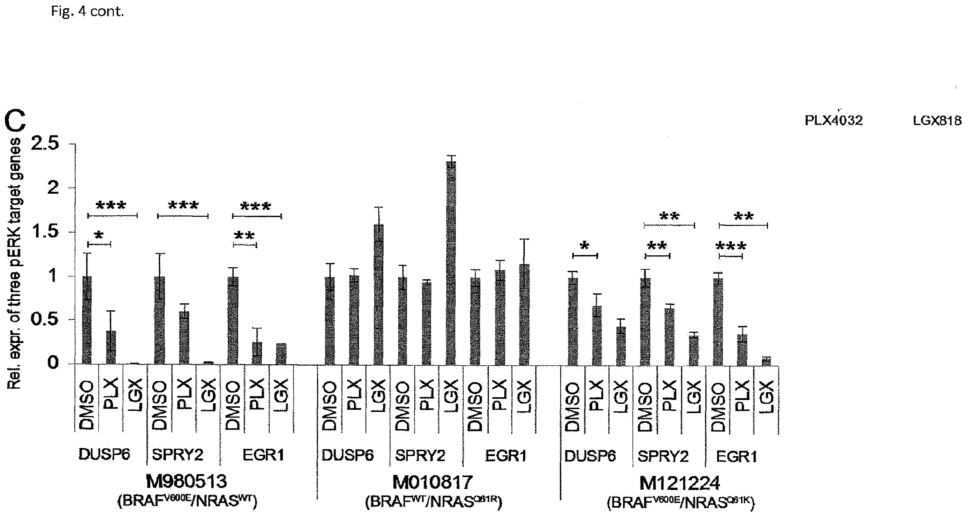

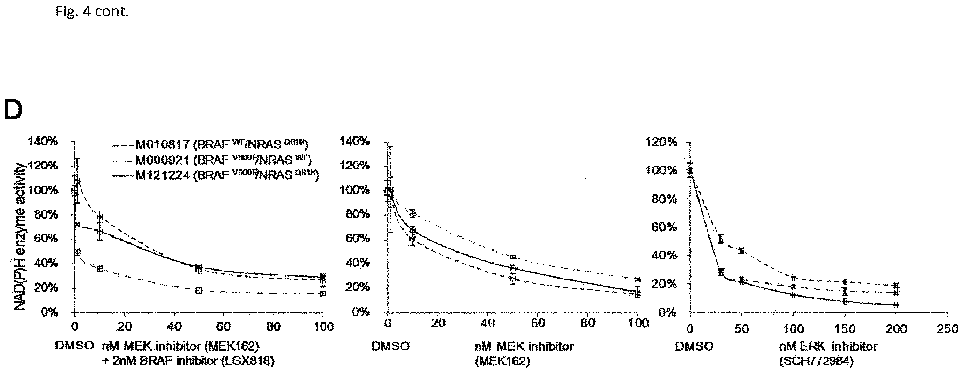

**Fig.4****:** Viability assays and pERK signaling in double-mutated melanoma cells. A resistant cell culture established from late metastasis 6 of patient 1 showed variable response to different BRAF inhibitors. (A) Triplicate MTT assays measuring NAD(P)H enzyme activity after treatment with different BRAF inhibitors normalized to DMSO treated cells. The resistant cell-line M121224, derived from a patient progressing while on LGX818 treatment, is fully resistant for LGX818, but only partially resistant to PLX4032 and GSK2118436. (B) Western blot and its Quantification of pERK levels in M121224 cells after BRAF-inhibitor treatment. Optical density of the bands was measured with ImageJ to obtain a bar-graph. Drug concentrations were chosen based on the IC50 of the sensitive cell-line M000921, as well as other BRAFV600E mutated early passage cultures. (C) qPCR showing the relative expression of pERK target genes after treatment with 0.35 µM PLX4032. (D) MTT assay measuring NAD(P)H enzyme activity after treatment with a MEK inhibitor (MEK162), a combination of MEK and BRAF inhibitor (LGX818) and ERK inhibitor (SCH772984) alone.

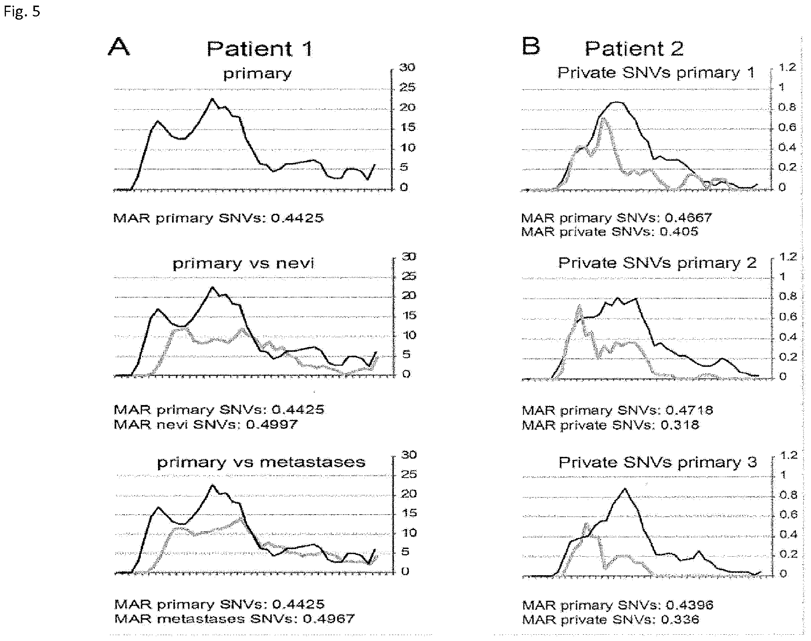

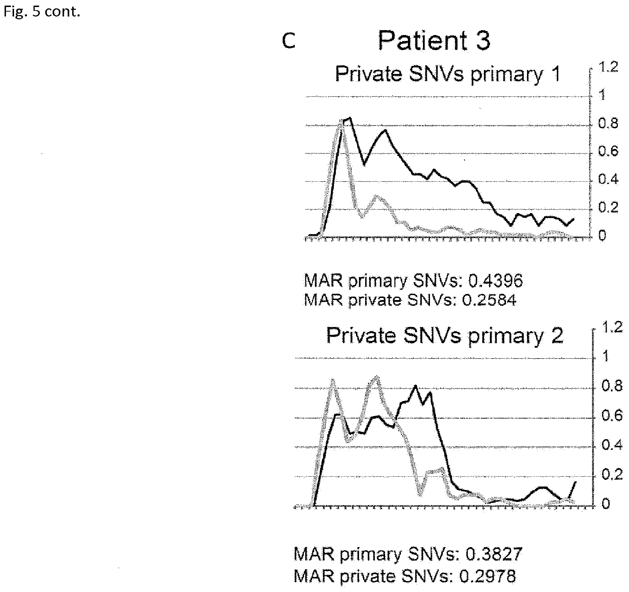

**Fig.5****:** Subclonal diversity measured by mutant allele ratios (MAR). (A) Frequencies of mutant allele ratios of the primary tumor of patient 1 show homozygous, heterozygous and possibly subclonal SNVs. A comparison to the nevi and metastases of patient 1 shows an increased subclonal frequency in the primary tumor. (B) Total SNVs of primary tumor of patient 2 (black line) compared to SNVs exclusively present in the first punch of the primary tumor of patient 2 (grey line). The SNVs private to the single punches generally have a low MAR. Values below the graphs represent mean MAR.

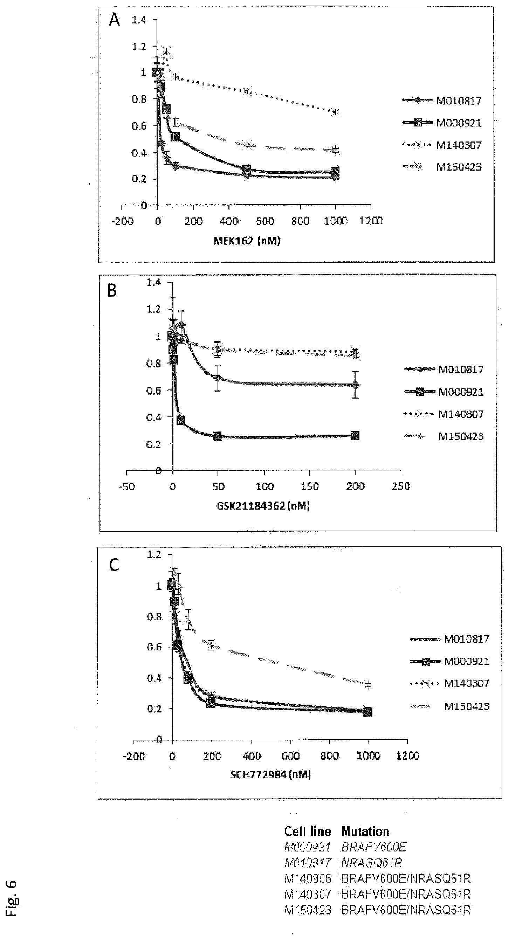

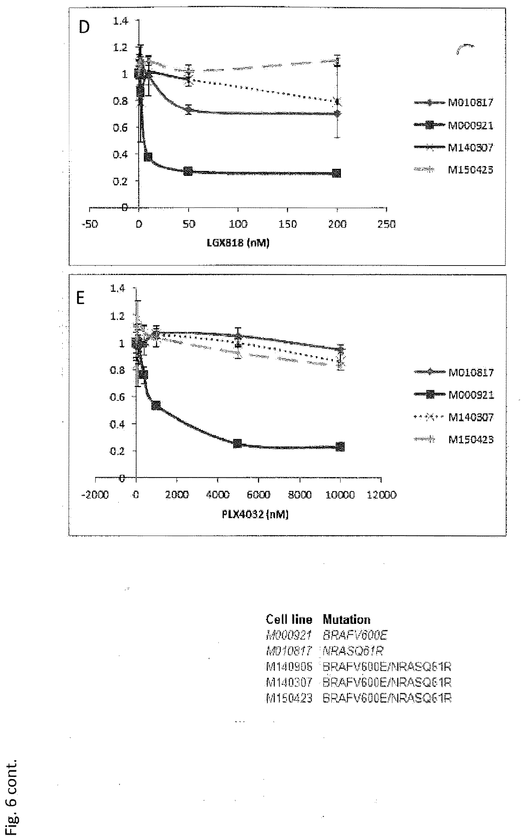

**Fig.6****:** Viability assays in double-mutated melanoma cells derived from single cell clones from metastatic melanoma. Triplicate MTT assays measuring NAD(P)H enzyme activity after treatment with the MEK inhibitor MEK162 (A), the ERK inhibitor SCH772984 (C) or the BRAF inhibitors GSK21184362 (B), LGX818 (D) or PLX4032 (E) normalized to DMSO treated cells. The BRAFV600E and NRASQ61R double mutated clonal cell-lines M140307 and M150423 are resistant to BRAF-inhibitor treatment, but sensitive to ERK inhibitor treatment.

### EXAMPLES

The Examples merely illustrate the invention or aspects thereof. They shall, by no means, interpreted as limiting the invention's scope.

The whole exome of multiple samples from three metastatic melanoma patients, which included diverse anatomical sites, therapies, and stages of disease progression (Figure 1 A-F) was seqenced. Patient 1 had a BRAFV600E mutation (Figure 1A), patient 2 had an unknown oncogenic driver (Figure 1B), and patient 3 had an activating NRASQ61R mutation (Figure 1C) at initial diagnosis. Patient 1 received a targeted BRAF inhibitor (i.e. LGX818) and had a partial response according to computed tomography (CT) (Figure 1D). Patient 2 progressed under multi-kinase inhibitor treatment i.e (i.e. pazopanib), according to PET/CT (Figure 1E). Patient 3 received a targeted MEK inhibitor (i.e. MEK162), and was also progressive according to CT (Figure 1F). Analysis of the sequencing results showed expected numbers of total single nucleotide variations (SNVs) in the tumor samples, as published in previous studies ^{6,12}. Both dysplastic nevi from patient 1 had a lower protein-coding mutational burden than any tumor biopsy from the three patients, as measured by the total number of genes with nonsynonymous SNVs. Nevus 1 had 133 and nevus 2 had 101 mutated genes, whereas patient 1's tumor biopsies had an average of 186 mutated genes. Patient 2 and patient 3 averaged 196 and 234 mutated genes in their tumors, respectively. Interestingly, in addition to having on average fewer numbers of mutated genes, the nevi had a reduced ratio of non-synonymous to synonymous mutations (i.e. 0.79) as compared to all other sequenced primary (1.20) and metastatic melanoma (1.22) lesions, indicating a lower proportion of protein coding changes in nevi versus melanoma tumors in general. It is also interesting to note that the primary tumors each had higher numbers of private SNVs than each patient's metastases, suggesting an increased exclusive genetic diversity in primary tumors than in metastases ¹³. For instance, patient 1 had 96 private SNVs exclusive to the primary tumor, and an average of 35 private SNVs in all metastases. Patient 2 had an average of 48 private SNVs exclusive to each of the three punches of the primary tumor, and on average 24 private SNVs in the metastases. Likewise, except for the one clear outlier metastasis (i.e. Late 1) in patient 3, each of the two primary tumor punches had higher numbers of private SNVs (i.e. 89) than the metastases (i.e. 38). Thus, overall the primary tumors had 2-2.7 fold significantly higher numbers (t-test, p<.00048) of private SNVs than the same patient's metastases in our cohort, with one outlier metastasis showing extraordinary numbers of private mutations.

Exome sequencing could confirm the known BRAF and NRAS mutation status that was initially identified by Sanger sequencing at the time of diagnosis for each patient (Figure 1). Additionally, the data for other known oncogenes and tumor suppressors were screened that could play a role in melanoma progression in our cohort. Although patient 2 had no known oncogenic drivers at the time of diagnosis, a non-synonymous germline mutation in the Melanocortin receptor MC1RV92M was identified, which has been shown to be significantly associated with an elevated risk of acquiring metastatic melanoma ¹⁴. In addition, patient 3 had the germline mutation MITFE318K that was recently associated with an increased risk of developing melanoma ¹⁵.

In order to identify genomic losses in potential tumor suppressor loci in these three patients, the exome data were analyzed with the EXCAVATOR and CONTRA algorithms ^{16,17}, which allowed to infer copy number variations (CNVs). A high number of CNVs could be detected in many chromosomes, with some samples exhibiting large losses throughout the genome (Figure 1 G-I).

Chromosomal imbalances could be identified in the investigated cohort that are known to occur frequently in melanoma (Figure 1 G-I). Patient 1 gained copies in 6p, 7, 8q and 17q (Figure 1G) in the late metastases 3 and 4 (Figure 1G). Patient 2 had gains in chromosome 1q, 7 and 22 in the late metastases (Figure 1H). In patient 3, we found gains in chromosome 1q, 6p and 20q (Figure 1I). All patients showed at least partial losses in chromosome 6q, 9p and 10 as well as in some samples in chromosome 11,2 and 17 (Figure 1 G-I).

In addition, CONTRA provides gene-specific information on CNVs. A consistent loss of the CDKN2A locus on chromosome 9 was found (Figure 1G-I) in all of the tumor samples, except in the nevi from patient 1. These losses were confirmed by qPCR to be homozygous in Patients 1 and 3, and heterozygous in patient 2 (data not shown), as predicted by both the EXCAVATOR and CONTRA algorithms (Figures 1G-I, suppl. Table 3). Furthermore, PTEN (chromosome 10) was lost in all samples of patient 2 (Figure 1H) and most of the samples from patient 1, except in the early met1 and the primary tumor.

One method to group tumor samples and build relationships between biopsies is to assume that CNVs, once lost, cannot be regained ¹⁸. Tumor phylogenies may thus be inferred by identifying specific genomic losses in a primary tumor, which cannot be recovered in a metastasis deriving from this primary. However, the high variability in intra-patient chromosomal imbalances that was identified would lead to many different possible relationships within the sampled biopsies (Figure 1 G-I). For example, in patient 3 the chromosome 10 CNVs would suggest that the late metastases derived from primary punch #2; however, the chromosome 14 CNVs are more suggestive of a late lineage deriving from primary punch #1 (Figure 1I) Likewise, in patient 2 the primary punch #2 has fewer losses in chromosome 11 than the other two primary punches, which suggests less similarity to the late metastases, whereas the pattern of losses on chromosome 3 would suggest a closer relationship between primary punch #2 and the late metastases (Figure 1 H). In general, intra-patient CNV heterogeneity was quite high, as can be observed in patients where we sequenced multiple regions of the same primary tumor (Figure 1 H,I). For example, in chromosome 11 of patient 2 and chromosomes 7, 10, 12 and 14 of patient 3, we found losses in only one of the two primary tumor punches. Heterogeneity in CNVs can also be clearly seen in patient 1 chromosome 22, for example, which has a predicted copy-number gain of the telomeric region in the primary tumor, which does not appear in any of the later metastases (Figure 1J).

### Example 2: Whole-exome phylogenetic analysis identifies inter-tumor relationships and progression-relevant SNVs

In order to investigate the evolutionary relationship between individual patient tumors in different therapeutic environments, phylogenetic algorithms were applied to the SNV and indel calls from each patient. Whole-exome phylogenetic analysis allowed to not only group tumor samples based on their total SNVs, insertions and deletions, but also to determine evolutionary relationships among the samples and to even find diagnostic characters supporting specific phylogenetic nodes (Figure 2). The biopsies from patient 1 and 3 (i.e. treated with BRAF and MEK targeted inhibitors, respectively) exhibited trees with post-resistance tumors forming monophyletic clades, meaning that all post resistant samples originated from only one node. Confidence is shown by bootstrap supports (arrow) which reflects the percentage of bootstrap trees also resolving the clade at the endpoints of that branch. Patient 2, who received non-targeted therapy (i.e. the multi-receptor tyrosine kinase inhibitor pazopanib) did not show this strong, monophyletic support of late tumor metastases (Figure 2) but the post resistant samples originated from multiple nodes (arrows).

The robust monophyletic topology of the phylogenetic trees from patient 1 and 3 upon targeted therapy suggest that the mechanism for therapeutic resistance may support the nodes that discriminate between the pre- and post-treatment clades (Figure 2 A, C). However, no known and shared mechanism of resistance to BRAF-inhibitor or MEK-inhibitor treatment could be identified in these node supports or in the whole-exome data that could explain the therapeutic resistance observed in patients 1 and 3. The intersection of non-synonymous SNVs between all post-relapse tumor exomes in each patient was investigated to find novel potential genetic resistance mechanisms. In patient 1 a somatic non-synonymous mutation in TACC1L452V was found that was ubiquitous and exclusive to the inhibitor-resistant tumor samples. Although TACC1 has been found to be frequently mutated in melanoma tumors, no role for TACC1 in treatment resistance has yet been identified 12 6. Since there may be intrapatient, inter-tumor heterogeneity of resistance mechanisms, it was sought to identify explanatory protein-coding changes in any of the post-treatment samples. In patient 1, a nonsynonymous mutation in GNAQT96S was detected in the primary and late metastasis 1, and TACC1C133A in the same biopsy. Although these mutations are in genes previously shown to be affected in melanoma, their role in treatment resistance remains unknown. Likewise, no known mechanisms of resistance were identified in the exome data of the other two patients.

### Example 3: Intra-patient genetic heterogeneity of LGX818 resistance

Given the lack of known, shared mechanisms of resistance in the two targeted therapy patients, the BRAF-inhibitor treated patient samples (i.e. patient 1) were further investigated, due to the greater knowledge of BRAF-inhibitor resistance mechanisms in the literature ⁹. Sanger sequencing was conducted on the same biopsy samples and on additional biopsies for which DNA was too limiting for exome sequencing without amplification. The BRAFV600E mutation could be confirmed by standard Sanger sequencing of PCR amplicons from all tumor samples (data not shown). Given that activating NRAS mutations are the most common resistance mechanism so far identified, being present in 17.8% of BRAF-inhibitor resistant tumors ⁹, it was chosen to first conduct Sanger sequencing of exons 2 and 3 of the NRAS locus in all patient 1 samples. In doing so, the activating mutation NRASQ61K in patient 1 late metastasis number 6 was identified which arose after relapse. The same mutation was absent in all other metastatic samples. Furthermore, it could be confirmed that this metastasis still had the BRAFV600E mutation, as well as two additional mutations that were found exclusively and ubiquitously in all of patient 1's other post-treatment metastases: TACC1L452V and C11orf30K22N (data not shown). No other specific mutations were tested by Sanger sequencing, but subsequent exome sequencing of a primary cell culture derived from late metastasis 6 (i.e. culture number M121224), could also confirm the presence of these mutations.

Since whole-exome sequencing provides broad genomic coverage, but limited depth at specific loci (in our case 101x average coverage across all samples), it is difficult to detect low-abundance subclones of cancer cells with alternative genotypes ^{19,20}. For this reason, digital PCR was applied to further investigate the possibility of a small subpopulation of mutated and resistant cells in patient 1's post-treatment tumors. Our digital PCR platform is based on 20'000 simultaneous PCR reactions per run, which allows for the detection of genomic variants present in as little as 5% of the tumor cell population.

By the use of this technique we measured the number of BRAFV600E or NRASQ61K mutated copies per microliter of DNA for each sample. Values with a precision of less than 15%, indicating a confidence interval of +/- 15% around the measured copy number, were considered acceptable. Digital PCR confirmed the presence of the BRAFV600E mutation in all tumors but not in DNA obtained from the patient's blood or nevus 1 (Figure 3A). Although late met 4 showed a low copy number per microliter, (i.e. 35 copies) the precision was within the acceptable range (i.e. 8.59%). However, all other tumor biopsies from patient 1, including those that had not been exome-sequenced (i.e. late metastases 5 & 6) had higher BRAFV600E copy numbers with good precision (Figure 3A). Also the presence of the NRASQ61K mutation in the late metastasis 6 was validated by digital PCR, and shown to have a high copy number in that metastasis (Figure 3A, green box). The digital PCR results also show the absence of detectable NRASQ61K subclones in any of the other resistant metastases aside from metastasis number 6 (Figure 3A).

### Example 4: Two activating MAPK mutations are present in single, BRAF-inhibitor resistant, but MEK and ERK-inhibitor sensitive melanoma cells

Although it could be show the presence of both MAPK-activation mutations BRAFV600E and NRASQ61K in a single post-resistance tumor from patient 1, these results may be explained by either the presence of two separate subpopulations of cells, each with one activating MAPK mutation, or the presence of both mutations in single cells. To distinguish between these possibilities, single melanoma cells were isolated from M121224 by FACS-sorting, and grew new cultures from each of these individual cells. Sanger sequencing of 26 cultures derived from 26 different single-cell clones could confirm the continued presence of both BRAFV600E and NRASQ61K mutations in all 23 independently derived colonies (Figure 3 B, C). To confirm that M121224 retained the BRAF inhibitor resistance of late metastasis 6, M121224 were treated with LGX818 and two other commercially available BRAF inhibitors (i.e. PLX4032 and GSK2128436), and cell viability was measured by the MTT assay (Figure 4A). A BRAFV600E mutated melanoma cell culture (M980513) was included as a positive control and an NRASQ61R mutated cell culture (M010817) as a negative control for BRAF inhibitor treatment. The M121224 line was still resistant to LGX818 to the same extent as the BRAFwt cell culture, M010817 (Figure 4A). Likewise, M121224 was also resistant to PLX4032 and GSK2118436 but to a lesser extent than the LGX818 inhibitor, to which the patient derived resistance (Figure 4A). Phosphorylated ERK (pERK) levels in M121224 were significantly decreased at the IC50 concentration of LGX818 and PLX4032 (Figure 4B). Significant down-regulation of three pERK target genes in M980513 and M121224 was observed at the IC50 concentration of PLX4032 and LGX818 (Figure 4C), but not in the control NRASQ61R cell line.

Although the M121224 double-mutated cells remained viable in the presence of high concentrations of the LGX818 drug (Figure 4A), there was curiosity how the co-existence of two activating MAPK mutations might affect the sensitivity of these cells to other MAPK pathway inhibitors. Treatment of M121224 cells with both the standard IC50 concentration of LGX818 and increasing concentrations of the MEK inhibitor (MEK162), could show viability profiles similar to cells with single NRASQ61R mutations (Figure 4D). Likewise, the MEK inhibitor alone was just as effective in reducing the viability of M121224 cells as it was with NRASQ61R mutated cells (Figure 4D). Finally, a specific ERK inhibitor alone also abrogated M121224 viability to the same degree as in BRAFV600E cells (Figure 4D).

The high sensitivity to ERK inhibitor treatment was further confirmed by experiments with additional double-mutated clonal cell lines isolated from BRAF-resistant metastatic melanoma (Figure 6).

### Example 5: Primary tumors exhibit highest subclonality

It is fair to assume that the majority of somatic mutations in cancer affect one but not the other allele and are thus heterozygous. In clonal and pure cancer population, such mutations demonstrate a mutant allele ratio (MAR) of 0.5, that is, half of all sequenced bases show the mutant allele. A deviation from this number may be indicative of the presence of cancer subclones, which give rise to a MARs smaller than 0.5. To study the presence of subclones in our primary tumors, the MARs across the multiple punches were determined. Moreover, d the mutant allele ratio (MAR) was calculated by dividing the number of mutant vs total read counts to get a measure of the potential presence of subclones within the samples ²¹. A higher proportion of SNVs with a low MAR was observed in the primary tumors vs nevi and metastases in patient 1, with the primary tumor having a mean MAR of 44%, while the nevi and metastases had mean MARs of 50% (Figure 5A). Furthermore, as the same primary tumor was punched multiple times, the MAR could be for all the samples of the primary tumors of patients 2 and 3. Presumably, these punches characterize different portions of the tumor, with some mutations found exclusively in on one punch, but not the other (private mutations). One must assume that these private mutations are subclonal, and are therefore present in a smaller set of cells. The results in Figure 5B clearly show that the mean MAR of the private SNVs of each primary tumor punch were considerably less than the overall MAR of all SNVs. The MAR of private vs total SNVs of each punch from patient two was between 6% and 16% less in each case (Figure 5B), and the mean MAR was between 9% and 15% less in the private SNVs of patient 3 than the total SNVs (Figure 5C). These results suggest that the primary tumors contain the highest subclonal diversity that can be characterized by a large number of private SNVs with low mutant allele frequencies. However, each primary tumor punch had different degrees of subclonality, suggesting heterogeneity in clonal diversity within tumors.

In patient 1, a bimodal distribution was observed of the MAFs in the primary tumor, with a peak at 0.35 and a secondary peak at 0.15. The first peak likely corresponds to clonal heterozygous mutations and indicates a tumor purity of 70%.

### Example 6: Experimental Procedures

### Sample preparation

Patient material was only used after written consent of the patient was given through the university biobank program according to ethical approval numbers 647 and 800. DNA was either isolated from paraffin embedded tissue stored in the biobank of the institute of Dermatology of the University Hospital of Zürich, fresh frozen tissue, or PBMCs. DNA from paraffin blocks was isolated using the FFPE DNA isolation kit from Qiagen (QIAamp DNA FFPE Tissue Kit #56404) and optimized protocols developed by Ultan McDermott at the Sanger institute. For DNA isolation from non-paraffin embedded samples we followed standard DNA isolation protocols published earlier. Given patient consent samples were collected during autopsy shortly after death. Samples were processed immediately after collection to ensure best possible DNA and RNA quality. Where possible, primary cell cultures were established as in previous studies ²⁸.

To reduce contamination with stromal tissue paraffin blocks were punched and the DNA was isolated out of the punches rather than from cuts of the whole block. Prior to DNA isolation, each tumor sample was evaluated by a trained dermato-histopathologist. Quality of the tissue as well as tumor content was checked and regions suitable for DNA isolation were marked. When available, DNA was sequenced from dysplastic nevi, primary melanoma tumors and metastases taken before therapy, as well as metastases obtained during necropsy. Germline DNA from PBMCs was sequenced for all patients if available as a reference ²⁹.

### Library preparation and sequencing

DNA quality was measured by an Agilent 2100 Bioanalyzer or Agilent 2200 Tapestation. One to three µg of high quality DNA was used to prepare the whole exome library using the Agilent SureSelect V4 or V5 kit. Sequencing was performed on an Illumina Hiseq 2000 machine in the Functional Genomics Center at University of Zürich. For the whole exome sequencing we sequenced 0.25 lanes per sample, paired-end, with 100bp reads.

### Whole exome sequencing analysis

Bioinformatics analysis was conducted with a modified GATK pipeline ³⁰⁻³²: Quality control was done with "FASTQC" ³³. Alignment of the FASTQ file to the reference genome "hg19" ³⁴ was done with "BWA" ³⁵. Transformation from SAM to BAM file format was done with "BWA". PCR duplicates were marked by MarkDuplicates from "Picard" ³⁶, Local realignment around indels with RealignerTargetCreator (GATK), realigning with IndelRealigner (GATK), fix mate information with FixMateInformation (Picard), base quality score recalibration with Baserecalibrator (GATK) and PrintReads (GATK). Variant calling was done with UnifiedGenotyper (GATK). For annotation of the VCF files we used Annovar ³⁷. Furthermore we used Samtools ³⁸ and Bedtools ³⁹. For data interpretation Microsoft Access, Microsoft Excel, Venny ⁴⁰, ConSet ⁴¹ and IGV ^{42,43} was used.

The mutant allele frequency was calculated for all the samples to get an impression of the degree of contamination with non-tumor tissue. Most of the samples showed a mutant allele frequency of 0.4 to 0.5 which corresponds to close to 100% tumor material being (Data not shown).

For copy number analysis we used Excavator 17 and Contra 16, results of the analysis with Excavator were visualized with Circos ⁴⁴.

SNVs were filtered according to the following read count criteria: A base must have at least four mutant reads and at least 10 total reads, if less than 10 total reads, at least half of them must be mutated. Also all SNVs with a phred-scaled quality score of <50 were excluded from further analysis. A SNV was called somatic if the unfiltered blood sample from the same patient did not show any mutant read for this position.

Mutant allele ratios (MAR) were calculated by dividing mutant read counts by total read counts for each called SNV. Frequencies for these ratios were calculated and trendlines were plotted in Excel with the Moving Average method (period: 3). To reduce the number of false positive SNVs more strict filtering was applied on the private SNVs. Quality threshold was raised to a phred score of 100, and the SNV needed to have at least 10 total reads. Genes that had more than 8 SNVs were excluded.

### dPCR

Digital PCR was carried out using the AB Gene Amp PCR System 9700 (Applied Biosystems Carlsbad, CA, USA), and with 15µl of the supplied mastermix (AB Quant Studio 3D) and equal amounts (0.6µM) of primers from Microsynth (Balgach, Switzerland).

| | | |
|---|---|---|
| BRAF | forward: 5'CTACTGTTTTCCTTTACTTACTACACCTCAGA | |
| | reverse: 5"ATCCAGACAACTGTTCAAACTGAT | |
| NRAS | forward: 5'GGTGAAACCTGTTTGTTGGACAT | |
| | reverse: 5'TGTATTGGTCTCTCATGGCACTGT | |

Additionally we used probes from Life Technologies (Carlsbad, CA, USA):

| | |
|---|---|
| BRAF V600E: | 6-VIC-TAGCTACAGAGAAATC-MGB |
| NRAS Q61K: | 6-FAM-CAGCTGGAAAAGAA-MGB |

The DNA was diluted to a final concentration of 4 µM; DNA concentration varied from 0.3ng/µl to 6.6ng/µl depending on the expected frequency of the target sequence. Chip loading and thermocycling conditions were according to the Life Technologies instructions. Fluorescence measurement was performed using the Quant Studio 3D and output was processed by QuantStudio 3D AnalysisSuite Software. Fluorescence values were Poisson corrected and copies per µl were calculated. Every sample showing a precision higher than 15% was classified as negative for the specific mutation.

### Sanger sequencing

After DNA amplification, 12ng of each PCR product, 5x Terminator Sequencing Buffer (Applied Biosystems), 1.5µM primers (Microsynth)

| | | |
|---|---|---|
| BRAF | forward: 5'CTAAGAGGAAAGATGAAGTACTATG | |
| | reverse: 5'CTAGTAACTCAGCAGCATCTCAG | |
| NRAS | forward: 5'GATAGGCAGAAATGGGCTTGA | |
| | reverse: 5'ATCATCCTTTCAGAGAAAATAATGC | |

and 2µl of BigDye Ready reaction Mix (Applied Biosystems) were added up to a 10µl reaction mix. Cycling conditions were performed as follows: 60s at 96°C were followed by 16 cycles for 10s at 96°C, 5s at 50°C and 240s at 60°C in a Lab Cycler (Sensoquest, Göttingen, Germany). Samples were purified using the Big Dye XTerminator purification Kit (Applied Biosystems) according to the manufacturer's manual. Subsequent Sanger Sequencing was carried out using the 3500 Genetic Analyzer (Applied Biosystems). Analysis was performed with the Variant Reporter Software (Life Technologies) where every mutation in the sequence which surpassed the threshold of 25% was classified as positive.

### Cell sorting

In order to perform single cell sorting of melanoma cells, the cells from a confluent T75 cell culture flask were pelleted and resuspended in 100µl FACS buffer (1% FBS, 5mM EDTA pH8, 0.01% NaN3/ddH2O in PBS). Cells were incubated for 20 minutes at 4°C with the following photosensitive antibodies: Anti-human MCSP-FITC (Miltenyi Biotec 130-098-794, Bergisch Gladbach Germany), diluted 1:20 in FACS buffer. Anti-human Fibroblasts/Epithelial-PE (ABIN319868, Aachen Germany), diluted 1:200 in FACS buffer. After washing, cells were resuspended in 200µl FACS buffer and sorted using the Aria IIb (BD Biosciences, Franklin Lakes, New Jersey, USA).

### Isolation of Melanoma cells from PBMCs

1x107 PBMCs were used for isolating melanoma cells with the CD56+CD16+NK cell isolation kit from Miltenyi Biotec (Bergisch Gladbach, Germany), according to the manufacturer's instructions. One deviation from the manual was in the last step, which is a positive selection for NK cells, whereas the flow-through contained the melanoma cells; other immune non-NK cells were depleted in the first step. After collecting the flow-through containing all non-immune cells, cells were pelleted for 5 minutes at 1500rpm and DNA isolation followed as with the non-paraffin samples reported here.

### Phylogenetic analysis:

Maximum Parsimony, Bayesian and Maximum likelihood (ML) phylogenies was constructed with the POSIX-threads version of RAxML v8.0.19 (7). An ascertainment bias correction and a general time-reversible (GTR) substitution model accounting for among-site rate heterogeneity using the Γ distribution and four rate categories (ASC_GTRGAMMA model) was used for calculation of the optimal tree. Node support was evaluated with 100 nonparametric bootstrap pseudoreplicates filtering the optimal ML tree through the bootstrap trees. Node support values therefore indicate the percent proportion of bootstrap trees that contained a given internode branch.

Variants diagnostic for a given clade are defined as existing solely in that clade and nowhere else for that position. All leaves emanating from the node in question must share a variant and all other leaves must contain a different character for a variant to be diagnostic. Diagnostic variants can therefore also be termed an apomorphy.

### Cell culture

Cell cultures were obtained from patient biopsies of cutaneous melanoma and melanoma metastasis after informed consent through the university biobank program according to ethical approval numbers 647 and 800. Tumor material was cut in small pieces and digested with 2.4U/ml Dispase (Roche, Basel, Switzerland) in RPMI1640 (Invitrogen (Carlsbad, CA, USA))for 3 hours at 37°C. Subsequently, the material was centrifuged (1500rpm/ 5min) and the supernatant was removed. Thereafter the pellet was dissolved in 0.005M Calcium Chloride dihydrate and 62.5U/ml Collagenase (Sigma, St. Louis, MO, USA) in Tris-buffered saline (pH 7.4) and incubated for 2 hours at 37°C. Subsequently, the material was centrifuged (1500rpm/ 5min) and the supernatant was removed. Stop solution (0.05M Tris Base, 0.15M NaCl and 0.01M EDTA in H2O, final pH 7.4) was added for 10 minutes. Thereafter, the pellet was washed two times with RPMI1640 and finally the cells were cultured in RPMI1640supplemented with 5mM L-glutamine (Biochrom, Berlin, Germany), 1mM sodium pyruvate (Gibco, Carlsbad, CA, USA) and 10% FCS (Gibco (Carlsbad, CA, USA)) in 37°C and 5% CO2 atmosphere. After several passages melanoma culture was confirmed by immunohistochemistry and mutation status of the cells was assessed.

### Cell viability assay

Cell sensitivity for different small molecule inhibitors was evaluated for the cell cultures M980513 (BRAFV600E, NRASWT), M000921 (BRAFV600E, NRASWT), M010817 (BRAFWT, NRASQ61R) and M121224 (BRAFV600E, NRASQ61K). 1x10^4 cells were seeded and treated for 72 hours with different concentrations of either a BRAF inhibitor (PLX4032, LGX818 or GSK2118436), a MEK inhibitor (MEK162), an ERK inhibitor (SCH772984), or a combination of a BRAF and MEK inhibitor (LGX818+MEK162). DMSO treatment was used as a control. After 72 hours, the medium was removed and fresh RPMI1640 supplemented with 10% FCS and 8% MTT reagent (Sigma, 5mg/ml in PBS) was added, and the cells were incubated at 37°C. After 1 hour, the RPMI1640 with MTT reagent was removed and 10% SDS (Sigma) and 95% isopropanol/ 5% Formic Acid (Sigma) (ratio 1:1) were added. After 5 min of incubation at 37°C, absorbance was measured at 595nm (reference 620nm) using a microplate reader.

### Western blot

Total protein was collected by washing cells twice with ice cold PBS and subsequent lysis in RIPA buffer (20mM Tris-HCl (pH 7.5), 1% Triton X-100 (Sigma), 137mM NaCl, 10% glycerol and protease inhibitors (Roche). Concentration of the protein was measured with the Bio-Rad Dc Protein Assay (Bio-Rad, Hercules, CA, USA) according to the manufacturer's protocol. SDS-Page was used to separate the proteins, after which they were transferred onto a nitrocellulose membrane. Membranes were probed with a rabbit anti-pERK antibody (Cell Signaling, product nr #4376S) and a rabbit anti-GAPDH antibody (Abcam, Cambridge, UK, product nr ab9385), followed by horseradish peroxidase-conjugated goat anti-rabbit IgG (Santa Cruz, product nr sc-2030)Bound antibodies were detected using chemiluminescence (ECL, GE Healthcare, Chalfont St. Giles, UK). Afterwards, band intensity was measured using ImageJ software (imagej.nih.gov/ij/) and pERK band intensity was corrected for corresponding GAPDH band intensity.

### qPCR analysis

Total RNA was extracted from cell cultures using TRIzol (Life Technologies), and afterwards 1µg of RNA was transcribed into cDNA with the Reverse Transcription System (A3500, Promega, Madison, WI, USA). For q-PCR, the ViiA7 (Life Technologies) was used, and the reaction mix consisted of 5µl SYBR Green (Roche), 3.5µl H2O, 0.5µl forward + reverse primer (10 µM) (Microsynth) and 1µl of cDNA (50 ng)
Cycling conditions were: 10min of 95°C, followed by 40 cycles of 95°C for 10 seconds and 58°C for 30 seconds, ending with 15 seconds of 95°C, 1 minute 60°C and 15 seconds 95°C. Gene expression differences of the pERK target genes DUSP6, SPRY2 and EGR1 (PMID19251651) were calculated using the ΔΔCT method. GAPDH was used as housekeeping gene.

**Primer Sequence**

| | | |
|---|---|---|
| GAPDH | Forward: | GAA GGT GAA GTT CGG AGT C |
| Reverse: | GAA GAT GGT GAT GGG ATT TC | |
| DUSP6 | Forward: | GAAATG GCG ATCAGC AAG ACG |
| Reverse: | CGA CGA CTC GTA TAG CTC CTG | |
| SPRY2 | Forward: | ATC AGA TCA GAG CCA TCC GAA |
| Reverse: | TGG AGT CTC TCG TGT TTG TGC | |
| EGR1 | Forward: | GGTCAGTGGCCTAGTGAGC |
| Reverse: | TGCTGTCGTTGGATGGCAC | |

### References cited through the application

1. Bollag G, Hirth P, Tsai J, et al. Clinical efficacy of a RAF inhibitor needs broad target blockade in BRAF-mutant melanoma. Nature. 2010;467(7315):596-599.
2. Chapman PB, Hauschild A, Robert C, et al. Improved survival with vemurafenib in melanoma with BRAF V600E mutation. N Engl J Med. 2011;364(26):2507-2516.
3. Flaherty KT, Puzanov I, Kim KB, et al. Inhibition of mutated, activated BRAF in metastatic melanoma. N Engl J Med. 2010;363(9):809-819.
4. Aplin AE, Kaplan FM, Shao Y. Mechanisms of resistance to RAF inhibitors in melanoma. J Invest Dermatol. 2011; 131(9):1817-1820.
5. Maley CC, Galipeau PC, Finley JC, et al. Genetic clonal diversity predicts progression to esophageal adenocarcinoma. Nat Genet. 2006;38(4):468-473.
6. Krauthammer M, Kong Y, Ha BH, et al. Exome sequencing identifies recurrent somatic RAC1 mutations in melanoma. Nat Genet. 2012;44(9):1006-1014.
7. Takata M, Morita R, Takehara K. Clonal heterogeneity in sporadic melanomas as revealed by loss-of-heterozygosity analysis. Int J Cancer. 2000;85(4):492-497.
8. Wilmott JS, Tembe V, Howle JR, et al. Intratumoral molecular heterogeneity in a BRAF-mutant, BRAF inhibitor-resistant melanoma: a case illustrating the challenges for personalized medicine. Mol Cancer Ther. 2012; 11(12):2704-2708.
9. Van Allen EM, Wagle N, Sucker A, et al. The genetic landscape of clinical resistance to RAF inhibition in metastatic melanoma. Cancer Discov. 2014;4(1):94-109.
10. Nazarian R, Shi H, Wang Q, et al. Melanomas acquire resistance to B-RAF(V600E) inhibition by RTK or N-RAS upregulation. Nature. 2010;468(7326):973-977.
11. Shi H, Hugo W, Kong X, et al. Acquired resistance and clonal evolution in melanoma during BRAF inhibitor therapy. Cancer Discov. 2014;4(1):80-93.
12. Hodis E, Watson IR, Kryukov GV, et al. A landscape of driver mutations in melanoma. Cell. 2012;150(2):251-263.
13. Nekrutenko A, Makova KD, Li WH. The K(A)/K(S) ratio test for assessing the protein-coding potential of genomic regions: an empirical and simulation study. Genome Res. 2002; 12(1): 198-202.
14. Fernandez L, Milne R, Bravo J, et al. MC1R: three novel variants identified in a malignant melanoma association study in the Spanish population. Carcinogenesis. 2007;28(8):1659-1664.
15. Berwick M, Macarthur J, Orlow I, et al. MITF E318K's effect on melanoma risk independent of, but modified by, other risk factors. Pigment Cell Melanoma Res. 2014;27(3):485-488.
16. Li J, Lupat R, Amarasinghe KC, et al. CONTRA: copy number analysis for targeted resequencing. Bioinformatics. 2012;28(10):1307-1313.
17. Magi A, Tattini L, Cifola I, et al. EXCAVATOR: detecting copy number variants from whole-exome sequencing data. Genome Biol. 2013;14(10):R120.
18. Schwarz RF, Trinh A, Sipos B, Brenton JD, Goldman N, Markowetz F. Phylogenetic Quantification of Intra-tumour Heterogeneity. PLoS Comput Biol. 2014;10(4):e1003535.
19. Flaherty P, Natsoulis G, Muralidharan O, et al. Ultrasensitive detection of rare mutations using next-generation targeted resequencing. Nucleic Acids Res. 2012;40(1):e2.
20. Gerstung M, Beisel C, Rechsteiner M, et al. Reliable detection of subclonal single-nucleotide variants in tumour cell populations. Nat Commun. 2012;3:811.
21. Nik-Zainal S, Van Loo P, Wedge DC, et al. The life history of 21 breast cancers. Cell. 2012;149(5):994-1007.
22. Guo J, Si L, Kong Y, et al. Phase II, open-label, single-arm trial of imatinib mesylate in patients with metastatic melanoma harboring c-Kit mutation or amplification. J Clin Oncol. 2011;29(21):2904-2909.
23. Ascierto PA, Schadendorf D, Berking C, et al. MEK162 for patients with advanced melanoma harbouring NRAS or Val600 BRAF mutations: a non-randomised, open-label phase 2 study. Lancet Oncol. 2013;14(3):249-256.
24. Emery CM, Vijayendran KG, Zipser MC, et al. MEK1 mutations confer resistance to MEK and B-RAF inhibition. Proc Natl Acad Sci U S A. 2009;106(48):20411-20416.
25. Wagle N, Emery C, Berger MF, et al. Dissecting therapeutic resistance to RAF inhibition in melanoma by tumor genomic profiling. J Clin Oncol. 2011;29(22):3085-3096.
26. Nissan MH, Pratilas CA, Jones AM, et al. Loss of NF1 in Cutaneous Melanoma Is Associated with RAS Activation and MEK Dependence. Cancer Res. 2014;74(8):2340-2350.
27. Poulikakos PI, Persaud Y, Janakiraman M, et al. RAF inhibitor resistance is mediated by dimerization of aberrantly spliced BRAF(V600E). Nature. 2011;480(7377):387-390.
28. Widmer DS, Cheng PF, Eichhoff OM, et al. Systematic classification of melanoma cells by phenotype-specific gene expression mapping. Pigment Cell Melanoma Res. 2012.
29. Böyum A. Isolation of mononuclear cells and granulocytes from human blood. Isolation of monuclear cells by one centrifugation, and of granulocytes by combining centrifugation and sedimentation at 1 g. Scand J Clin Lab Invest Suppl. 1968;97:77-89.
30. McKenna A, Hanna M, Banks E, et al. The Genome Analysis Toolkit: a MapReduce framework for analyzing next-generation DNA sequencing data. Genome Res. 2010;20(9):1297-1303.
31. Van der Auwera GA. From FastQ Data to High-Confidence Variant Calls: The Genome Analysis Toolkit Best Practices Pipeline. In: G. A. C, ed. Current Protocols in Bioinformatics: Wiley Online Library; 2013.
32. DePristo MA, Banks E, Poplin R, et al. A framework for variation discovery and genotyping using next-generation DNA sequencing data. Nat Genet. 2011;43(5):491-498.
33. Andrews S. FastQC A Quality Control tool for High Throughput Sequence Data. http://www.bioinformatics.babraham.ac.uk/projects/fastqc/.
34. Lander ES, Linton LM, Birren B, et al. Initial sequencing and analysis of the human genome. Nature. 2001;409(6822):860-921.
35. Li H, Durbin R. Fast and accurate short read alignment with Burrows-Wheeler transform. Bioinformatics. 2009;25(14): 1754-1760.
36. Picard. http://picard.sourceforge.net/.
37. Wang K, Li M, Hakonarson H. ANNOVAR: functional annotation of genetic variants from high-throughput sequencing data. Nucleic Acids Res. 2010;38(16):e164.
38. Li H, Handsaker B, Wysoker A, et al. The Sequence Alignment/Map format and SAMtools. Bioinformatics. 2009;25(16):2078-2079.
39. Quinlan AR, Hall IM. BEDTools: a flexible suite of utilities for comparing genomic features. Bioinformatics. 2010;26(6):841-842.
40. Oliveros JC. VENNY. An interactive tool for comparing lists with Venn diagrams. 2007; http://bioin-fogp.cnb.csic.es/tools/venny/index.html.
41. Kim B, Lee B, Seo J. Visualizing Set Concordance with Permutation Matrices and Fan Diagrams. Interact Comput. 2007;19(5):630-643.
42. Thorvaldsdóttir H, Robinson JT, Mesirov JP. Integrative Genomics Viewer (IGV): high-performance genomics data visualization and exploration. Brief Bioinform. 2013;14(2):178-192.
43. Robinson JT, Thorvaldsdóttir H, Winckler W, et al. Integrative genomics viewer. Nat Biotechnol. 2011;29(1):24-26.
44. Krzywinski M, Schein J, Birol I, et al. Circos: an information aesthetic for comparative genomics. Genome Res. 2009;19(9):1639-1645.
45. Sensi, M., Nicolini, G., Petti, C., et al. Mutually exclusive NRASQ61R and BRAFV600E mutations at the single-cell level in the same human melanoma. Oncogene, 2006, 25(24), 3357-3364.
46. Thumar, J et al. (2014). MEK targeting in N-RAS mutated metastatic melanoma. Molecular cancer, 13(1), 45.
47. Greger, J. G et al. (2012). Combinations of BRAF, MEK, and PI3K/mTOR inhibitors overcome acquired resistance to the BRAF inhibitor GSK2118436 dabrafenib, mediated by NRAS or MEK mutations. Molecular cancer therapeutics, 11(4), 909-920.
48. Romano, E. et al. (2013). Identification of multiple mechanisms of resistance to vemurafenib in a patient with BRAFV600E-mutated cutaneous melanoma successfully rechallenged after progression. Clinical Cancer Research, 19(20), 5749-5757.
49. Kaplan, F. M. et al.. (2012). SHOC2 and CRAF mediate ERK1/2 reactivation in mutant NRAS-mediated resistance to RAF inhibitor. Journal of Biological Chemistry, 287(50), 41797-41807.
50. WO 2012/068562 A2
51. WO 2012/068468 A1
52. US 2014/018405 A1
53. US 2014/018372 A1
54. WO 2012/058532 A2
55. WO 2014/052613 A2

## Claims

1. A method for identifying whether a subject suffering from a BRAF-positive cancer, wherein said BRAF-positive cancer is comprised of a cell population derived from a single cell clone, is a non-responder to a BRAF inhibitor, or not, and/or is a responder to an MAPK/ERK inhibitor comprising the steps of:
(a) determining the presence or absence of at least one mutation in at least the NRAS gene in a sample of the subject; wherein said sample is derived from a single cell clone, and
(b) identifying the subject as a non-responder to a BRAF inhibitor and a responder to a MAPK/ERK inhibitor if the at least one mutation in the NRAS gene has been determined.

2. The method of claim 1, wherein said method further comprises determining the presence or absence of at least one mutation in the BRAF gene, whereby the presence of the said at least one mutation further identifies the subject as a non-responder to a BRAF inhibitor and a responder to a MAPK/ERK inhibitor.

3. The method of claim 1 or 2, wherein the BRAF-positive cancer is melanoma cancer.

4. The method of any ones of claims 1 to 3, wherein the cells of the cell population derived from a single cell clone contain in their genome at least one mutation in the BRAF gene and at least one mutation in the NRAS gene.

5. The method of any one of claims 1 to 4, wherein the BRAF-inhibitor is a small molecule inhibitor of BRAF activity and, preferably, LGX818, PLX4032 and/or GSK2118436.

6. The method of any of claims 1 to 5, wherein the said MAPK/ERK inhibitor is a small molecule inhibitor of MEK or an ERK activity and, preferably, GSK1120212, MEK162, and said inhibitor of ERK activity is SCH772984.

7. The method of any of claims 1 to 6, wherein the mutation of the NRAS gene results in an amino acid substitution at a position corresponding to amino acid 61 of exon 2 of the human NRAS protein.

8. The method of claim 7, wherein said amino acid substitution is a glutamine-to-lysine substitution (Q61K), a glutamine-to-arginine substitution (Q61R), or a glutamine-to-leucine (Q61L).

9. The method of any of claims 2 to 8, wherein the mutation of the BRAF gene results in an amino acid substitution at a position corresponding to amino acid 600 of exon 15 of human BRAF protein.

10. The method of claim 9, wherein said amino acid substitution is a valine-to-glutamate substitution (V600E), a valine-to-lysine substitution (V600K), a valine-to-arginine substitution (V600R), or a valine-to-aspartic acid substitution (V600D).

11. The method of any one of claims 1 to 10, wherein the presence of the at least one mutation in exon 2 of the catalytic subunit of NRAS nucleic acid is determined by
a) contacting nucleic acids in the sample from the subject with one or more of the locus-specific oligonucleotides selected from the group consisting of: GGTGAAACCTGTTTGTTGGACAT (SEQ ID NO:7); TGTATTGGTCTCTCATGGCACTGT (SEQ ID NO:8); GATAGGCAGAAATGGGCTTGA (SEQ ID NO:9); and ATCATCCTTTCAGAGAAAATAATGC (SEQ ID NO:10);
b) incubating the sample under conditions allowing specific hybridization of the oligonucleotide to its target sequence within a NRAS nucleic acid;
c) detecting said hybridization; and
d) determining the at least one mutation based on said hybridization detected in step c).

12. The method of claim 11, wherein step b) further comprises the step of generating an amplification product containing the target sequence within the NRAS nucleic acid by amplifying the NRAS nucleic acid in the sample with one or both of the following oligonucleotide primers: forward oligonucleotide primer having SEQ ID NO:11 and reverse oligonucleotide primer having SEQ ID NO:12.

13. The method of any one of claims 2 to 12, wherein the presence of the at least one mutation in exon 15 of the catalytic subunit of BRAF nucleic acid is determined by
a) contacting nucleic acids in the sample from the subject with one or more of the locus-specific oligonucleotides selected from the group consisting of: CTAAGAGGAAAGATGAAGTACTATG (SEQ ID NO:1); CTAGTAACTCAGCAGCATCTCAG (SEQ ID NO:2); CTACTGTTTTCCTTTACTTACTACACCTCAGA (SEQ ID NO:3); and ATCCAGACAACTGTTCAAACTGAT(SEQ ID NO:4);
b) incubating the sample under conditions allowing specific hybridization of the oligonucleotide to its target sequence within a BRAF nucleic acid;
c) detecting said hybridization; and
d) determining the at least one mutation based on said hybridization detected in step c).

14. The method of claim 13, wherein step b) further comprises the step of generating an amplification product containing the target sequence within the BRAF nucleic acid by amplifying the NRAS nucleic acid in the sample with one or both of the following oligonucleotide primers: forward oligonucleotide primer having SEQ ID NO:5 and reverse oligonucleotide primer having SEQ ID 6.

15. The method of any one of claims 1 to 14, wherein said method further comprises recommending to the subject the administration of a MAPK/ERK inhibitor drug if the subject has been identified as a non-responder to a BRAF inhibitor and a responder to a MAPK/ERK inhibitor.

16. An MAPK/ERK inhibitor for use in treating a subject suffering from a BRAF-positive cancer wherein said BRAF-positive cancer is comprised of a cell population derived from a single cell clone, whereby the said cancer has been found to comprise cancer cells carrying at least one mutation in the NRAS gene and at least one mutation in the BRAF gene.

17. A method of assessing responsiveness to targeted therapy in a subject comprising:
a) providing a sample from the subject, wherein said sample is derived from a single cell clone;
b) testing the sample for the presence of mutations in the BRAF and the NRAS genes, wherein testing is performed by one of the methods selected from a group consisting of selective amplification, probe hybridization or nucleic acid sequencing
c) if mutations in the NRAS and BRAF genes are detected, detecting responsiveness to a MAPK/ERK inhibitor and non-responsiveness to a BRAF inhibitor.

18. A method of assessing cancer in a subject comprising:
a) providing a sample from the subject containing nucleic acids wherein said sample is derived from a single cell clone,
b) contacting the nucleic acids in the sample with a nucleic acid probe specific for mutations in the BRAF and NRAS genes,
c) if mutations in the NRAS and BRAF genes are detected, assessing the cancer as responsive to a MAPK/ERK inhibitor and non-responsive to a BRAF inhibitor.

## Patentansprüche

1. Verfahren zur Identifizierung, ob ein an einer BRAF-positiven Krebserkrankung leidendes Individuum, wobei der BRAF-positive Krebs aus einer von einem einzelnen Zellklon stammenden Zellpopulation besteht, nicht auf einen BRAF-Inhibitor anspricht oder doch und/oder auf einen MAPK/ERK-Inhibitor anspricht, umfassend die Schritte:
(a) Bestimmen des Vorliegens oder Fehlens wenigstens einer Mutation in wenigstens dem NRAS-Gen in einer Probe vom Individuum, wobei die Probe von einem einzelnen Zellklon stammt, und
(b) Identifizieren des Individuums als nicht auf einen BRAF-Inhibitor ansprechend und auf einen MAPK/ERK-Inhibitor ansprechend, falls die wenigstens eine Mutation im NRAS-Gen bestimmt wurde.

2. Verfahren nach Anspruch 1, wobei das Verfahren ferner Bestimmen des Vorliegens oder Fehlens wenigstens einer Mutation im BRAF-Gen umfasst, wobei durch das Vorliegen der wenigstens einen Mutation das Individuum weiter als nicht auf einen BRAF-Inhibitor ansprechend und auf einen MAPK/ERK-Inhibitor ansprechend identifiziert wird.

3. Verfahren nach Anspruch 1 oder 2, wobei es sich bei der BRAF-positiven Krebserkrankung um Melanom-Krebs handelt.

4. Verfahren nach einem der Ansprüche 1 bis 3, wobei die Zellen der von einem einzelnen Zellklon stammenden Zellpopulation in ihrem Genom wenigstens eine Mutation im BRAF-Gen und wenigstens eine Mutation im NRAS-Gen enthalten.

5. Verfahren nach einem der Ansprüche 1 bis 4, wobei es sich bei dem BRAF-Inhibitor um einen niedermolekularen Inhibitor der BRAF-Aktivität und bevorzugt um LGX818, PLX4032 und/oder GSK2118436 handelt.

6. Verfahren nach einem der Ansprüche 1 bis 5, wobei es sich bei dem MAPK/ERK-Inhibitor um einen niedermolekularen Inhibitor von MEK- oder einer ERK-Aktivität und bevorzugt um GSK1120212, MEK162 und bei dem Inhibitor von ERK-Aktivität um SCH772984 handelt.

7. Verfahren nach einem der Ansprüche 1 bis 6, wobei die Mutation des NRAS-Gens zu einer Aminosäuresubstitution an einer Aminosäure 61 von Exon 2 des menschlichen NRAS-Proteins entsprechenden Position führt.

8. Verfahren nach Anspruch 7, wobei es sich bei der Aminosäuresubstitution um eine Glutamin-gegen-Lysin-Substitution (Q61K), eine Glutamin-gegen-Arginin-Substitution (Q61R) oder eine Glutamingegen-Leucin (Q61L) handelt.

9. Verfahren nach einem der Ansprüche 2 bis 8, wobei die Mutation des BRAF-Gens zu einer Aminosäuresubstitution an einer Aminosäure 600 von Exon 15 des menschlichen BRAF-Proteins entsprechenden Position führt.

10. Verfahren nach Anspruch 9, wobei es sich bei der Aminosäuresubstitution um eine Valin-gegen-Glutamat-Substitution (V600E), eine Valin-gegen-Lysin-Substitution (V600K), eine Valin-gegen-Arginin-Substitution (V600R) oder eine Valingegen-Asparaginsäure-Substitution (V600D) handelt.

11. Verfahren nach einem der Ansprüche 1 bis 10, wobei das Vorliegen der wenigstens einen Mutation in Exon 2 der katalytischen Untereinheit von NRAS-Nukleinsäure bestimmt wird durch
(a) Inkontaktbringen von Nukleinsäuren in der Probe vom Individuum mit einem oder mehreren der locus-spezifischen Oligonukleotide ausgewählt aus der Gruppe bestehend aus: GGTGAAACCTGTTTGTTGGACAT (SEQ ID NO:7); TGTATTGGTCTCTCATGGCACTGT (SEQ ID NO:8); GATAGGCAGAAATGGGCTTGA (SEQ ID NO:9); und ATCATCCTTTCAGAGAAAATAATGC (SEQ ID NO:10);
(b) Inkubieren der Probe unter Bedingungen, die eine spezifische Hybridisierung des Oligonukleotids an seine Zielsequenz in einer NRAS-Nukleinsäure gestatten;
(c) Nachweisen der Hybridisierung; und
(d) Bestimmen der wenigstens einen Mutation basierend auf der in Schritt c) nachgewiesenen Hybridisierung.

12. Verfahren nach Anspruch 11, wobei Schritt b) ferner den Schritt Erzeugen eines Amplifikationsprodukts, das die Zielsequenz in der NRAS-Nukleinsäure enthält, durch Amplifizieren der NRAS-Nukleinsäure in der Probe mit einem oder beiden der folgenden Oligonukleotidprimer: Vorwärts-Oligonukleotidprimer mit SEQ ID NO: 11 und Rückwärts-Oligonukleotidprimer mit SEQ ID NO: 12, umfasst.

13. Verfahren nach einem der Ansprüche 2 bis 12, wobei das Vorliegen der wenigstens einen Mutation in Exon 15 der katalytischen Untereinheit von BRAF-Nukleinsäure bestimmt wird durch
(a) Inkontaktbringen von Nukleinsäuren in der Probe vom Individuum mit einem oder mehreren der locus-spezifischen Oligonukleotide ausgewählt aus der Gruppe bestehend aus: CTAAGAGGAAAGATGAAGTACTATG (SEQ ID NO:1); CTAGTAACTCAGCAGCATCTCAG (SEQ ID NO:2); CTACTGTTTTCCTTTACTTACTACACCTCAGA (SEQ ID NO:3); und ATCCAGACAACTGTTCAAACTGAT (SEQ ID NO:4;
(b) Inkubieren der Probe unter Bedingungen, die eine spezifische Hybridisierung des Oligonukleotids an seine Zielsequenz in einer BRAF-Nukleinsäure gestatten;
(c) Nachweisen der Hybridisierung; und
(d) Bestimmen der wenigstens einen Mutation basierend auf der in Schritt c) nachgewiesenen Hybridisierung.

14. Verfahren nach Anspruch 13, wobei Schritt b) ferner den Schritt Erzeugen eines Amplifikationsprodukts, das die Zielsequenz in der BRAF-Nukleinsäure enthält, durch Amplifizieren der NRAS-Nukleinsäure in der Probe mit einem oder beiden der folgenden Oligonukleotidprimer: Vorwärts-Oligonukleotidprimer mit SEQ ID NO:5 und Rückwärts-Oligonukleotidprimer mit SEQ ID 6, umfasst.

15. Verfahren nach einem der Ansprüche 1 bis 14, wobei das Verfahren ferner umfasst, dem Individuum die Verabreichung eines MAPK/ERK-Inhibitor-Arzneistoffs zu empfehlen, falls das Individuum als nicht auf einen BRAF-Inhibitor ansprechend und auf einen MAPK/ERK-Inhibitor ansprechend identifiziert wurde.

16. MAPK/ERK-Inhibitor zur Verwendung bei der Behandlung eines an einer BRAF-positiven Krebserkrankung leidenden Individuums, wobei der BRAF-positive Krebs aus einer von einem einzelnen Zellklon stammenden Zellpopulation besteht, wobei festgestellt wurde, dass der Krebs Krebszellen umfasst, die wenigstens eine Mutation im NRAS-Gen und wenigstens eine Mutation im BRAF-Gen tragen.

17. Verfahren zur Beurteilung der Ansprechbarkeit auf eine gezielte Therapie bei einem Individuum, umfassend:
(a) Bereitstellen einer Probe von dem Individuum, wobei die Probe von einem einzelnen Zellklon stammt,
(b) Testen der Probe auf das Vorliegen von Mutationen im BRAF- und NRAS-Gen, wobei das Testen mit einer der aus einer Gruppe bestehend aus selektiver Amplifikation, Sondenhybridisierung oder Nukleinsäuresequenzierung ausgewählten Methoden erfolgt,
(c) falls Mutationen im NRAS- und BRAF-Gen nachgewiesen werden, Nachweisen der Ansprechbarkeit auf einen MAPK/ERK-Inhibitor und Nichtansprechbarkeit auf einen BRAF-Inhibitor.

18. Verfahren zur Beurteilung von Krebs bei einem Individuum, umfassend:
(a) Bereitstellen einer Nukleinsäuren enthaltenden Probe von dem Individuum, wobei die Probe von einem einzelnen Zellklon stammt;
(b) Inkontaktbringen der Nukleinsäuren in der Probe mit einer Nukleinsäuresonde, die für Mutationen im BRAF- und NRAS-Gen spezifisch ist,
(c) falls Mutationen im NRAS- und BRAF-Gen nachgewiesen werden, Beurteilen des Krebses als ansprechend auf einen MAPK-ERK-Inhibitor und nicht ansprechend auf einen BRAF-Inhibitor.

## Revendications

1. Procédé permettant d'identifier si un sujet souffrant d'un cancer BRAF-positif, ledit cancer BRAF-positif étant constitué d'une population de cellules dérivée d'un clone monocellulaire, étant un non-répondeur à un inhibiteur de BRAF, ou non, et/ou étant un répondeur à un inhibiteur de MAPK/ERK comprenant les étapes de :
(a) détermination de la présence ou de l'absence d'au moins une mutation dans au moins le gène NRAS dans un échantillon du sujet ; et ledit échantillon étant dérivé d'un clone monocellulaire
(b) identification du sujet comme non-répondeur à un inhibiteur de BRAF et répondeur à un inhibiteur de MAPK/ERK si l'au moins une mutation dans le gène NRAS a été déterminée.

2. Procédé selon la revendication 1, dans lequel ledit procédé comprend en outre la détermination de la présence ou de l'absence d'au moins une mutation dans le gène BRAF, moyennant quoi la présence de ladite au moins une mutation identifie en outre le sujet comme non-répondeur à un inhibiteur de BRAF et répondeur à un inhibiteur de MAPK/ERK.

3. Procédé selon la revendication 1 ou 2, dans lequel le cancer BRAF-positif est un cancer de type mélanome.

4. Procédé selon l'une quelconque des revendications 1 à 3, dans lequel les cellules de la population de cellules dérivée d'un clone monocellulaire contiennent dans leur génome au moins une mutation dans le gène BRAF et au moins une mutation dans le gène NRAS.

5. Procédé selon l'une quelconque des revendications 1 à 4, dans lequel l'inhibiteur de BRAF est un inhibiteur à petite molécule de l'activité BRAF et, de préférence, LGX818, PLX4032 et/ou GSK2118436.

6. Procédé selon l'une quelconque des revendications 1 à 5, dans lequel ledit inhibiteur de MAPK/ERK est un inhibiteur à petite molécule de l'activité MEK ou ERK et, de préférence, GSK1120212, MEK162, et ledit inhibiteur de l'activité ERK est SCH772984.

7. Procédé selon l'une quelconque des revendications 1 à 6, dans lequel la mutation du gène NRAS conduit à une substitution d'acide aminé au niveau d'une position correspondant à l'acide aminé 61 de l'exon 2 de la protéine NRAS humaine.

8. Procédé selon la revendication 7, dans lequel ladite substitution d'acide aminé est une substitution de glutamine en lysine (Q61K), une substitution de glutamine en arginine (Q61R) ou une substitution de glutamine en leucine (Q61L).

9. Procédé selon l'une quelconque des revendications 2 à 8, dans lequel la mutation du gène BRAF conduit à une substitution d'acide aminé au niveau d'une position correspondant à l'acide aminé 600 de l'exon 15 de la protéine BRAF humaine.

10. Procédé selon la revendication 9, dans lequel ladite substitution d'acide aminé est une substitution de valine en glutamate (V600E), une substitution de valine en lysine (V600K), une substitution de valine en arginine (V600R) ou une substitution de valine en acide aspartique (V600D).

11. Procédé selon l'une quelconque des revendications 1 à 10, dans lequel la présence de l'au moins une mutation dans l'exon 2 de la sous-unité catalytique de l'acide nucléique NRAS est déterminée par
(a) mise en contact d'acides nucléiques dans l'échantillon du sujet avec un ou plusieurs des oligonucléotides spécifiques d'un locus choisis dans le groupe constitué par : GGTGAAACCTGTTTGTTGGACAT (SEQ ID NO : 7) ; TGTATTGGTCTCTCATGGCACTGT (SEQ ID NO : 8) ; GATAGGCAGAAATGGGCTTGA (SEQ ID NO : 9) ; et ATCATCCTTTCAGAGAAAATAATGC (SEQ ID NO : 10) ;
(b) incubation de l'échantillon dans des conditions permettant l'hybridation spécifique de l'oligonucléotide à sa séquence cible dans un acide nucléique NRAS ;
(c) détection de ladite hybridation ; et
(d) détermination de l'au moins une mutation sur la base de ladite hybridation détectée à l'étape c).

12. Procédé selon la revendication 11, dans lequel l'étape b) comprend en outre l'étape de génération d'un produit d'amplification contenant la séquence cible dans l'acide nucléique NRAS par amplification de l'acide nucléique NRAS dans l'échantillon avec l'une ou les deux des amorces oligonucléotidiques suivantes : amorce oligonucléotidique sens ayant la SEQ ID NO : 11 et amorce oligonucléotidique antisens ayant la SEQ ID NO : 12.

13. Procédé selon l'une quelconque des revendications 2 à 12, dans lequel la présence de l'au moins une mutation dans l'exon 15 de la sous-unité catalytique de l'acide nucléique BRAF est déterminée par
(a) mise en contact d'acides nucléiques dans l'échantillon du sujet avec un ou plusieurs des oligonucléotides spécifiques d'un locus choisis dans le groupe constitué par : CTAAGAGGAAAGATGAAGTACTATG (SEQ ID NO : 1) ; CTAGTAACTCAGCAGCATCTCAG (SEQ ID NO : 2) ; CTACTGTTTTCCTTTACTTACTACACCTCAGA (SEQ ID NO : 3) ; et ATCCAGACAACTGTTCAAACTGAT(SEQ ID NO : 4) ;
(b) incubation de l'échantillon dans des conditions permettant l'hybridation spécifique de l'oligonucléotide à sa séquence cible dans un acide nucléique BRAF ;
(c) détection de ladite hybridation ; et
(d) détermination de l'au moins une mutation sur la base de ladite hybridation détectée à l'étape c).

14. Procédé selon la revendication 13, dans lequel l'étape b) comprend en outre l'étape de génération d'un produit d'amplification contenant la séquence cible dans l'acide nucléique BRAF par amplification de l'acide nucléique NRAS dans l'échantillon avec l'une ou les deux des amorces oligonucléotidiques suivantes : amorce oligonucléotidique sens ayant la SEQ ID NO : 5 et amorce oligonucléotidique antisens ayant la SEQ ID NO : 6.

15. Procédé selon l'une quelconque des revendications 1 à 14, dans lequel ledit procédé comprend en outre la recommandation au sujet de l'administration d'un médicament inhibiteur de MAPK/ERK si le sujet a été identifié comme étant un non-répondeur à un inhibiteur de BRAF et un répondeur à un inhibiteur de MAPK/ERK.

16. Inhibiteur de MAPK/ERK pour une utilisation dans le traitement d'un sujet souffrant d'un cancer BRAF-positif, dans lequel ledit cancer BRAF-positif est constitué d'une population de cellules dérivée d'un clone monocellulaire, moyennant quoi ledit cancer a été déterminé comme comprenant des cellules cancéreuses portant au moins une mutation dans le gène NRAS et au moins une mutation dans le gène BRAF.

17. Procédé d'évaluation de la réponse à une thérapie ciblée chez un sujet comprenant :
(a) la fourniture d'un échantillon du sujet, ledit échantillon étant dérivé d'un clone monocellulaire ;
(b) l'analyse de l'échantillon pour la présence de mutations dans les gènes BRAF et NRAS, l'analyse étant effectuée par l'un des procédés choisis dans un groupe constitué par l'amplification sélective, l'hybridation de sonde ou le séquençage d'acide nucléique
(c) si des mutations dans les gènes NRAS et BRAF sont détectées, la détection de la réponse à un inhibiteur de MAPK/ERK et de la non-réponse à un inhibiteur de BRAF.

18. Procédé d'évaluation d'un cancer chez un sujet comprenant :
(a) la fourniture d'un échantillon du sujet contenant des acides nucléiques, ledit échantillon étant dérivé d'un clone monocellulaire,
(b) la mise en contact des acides nucléiques dans l'échantillon avec une sonde d'acide nucléique spécifique pour des mutations dans les gènes BRAF et NRAS,
(c) si des mutations dans les gènes NRAS et BRAF sont détectées, l'évaluation du cancer comme étant répondeur à un inhibiteur de MAPK/ERK et non-répondeur à un inhibiteur de BRAF.


### SEQUENCE LISTING

<110> Universitat Zürich
<120> Means and methods for identifying a patient having a BRAF-positive cancer as a non-responder to a BRAF inhibitor and as a responder to an MAPK/ERK inhibitor
<130> RD32112EP
<160> 28
<170> PatentIn version 3.3
<210> 1
   <211> 25
   <212> DNA
   <213> Homo sapiens
<400> 1
   ctaagaggaa agatgaagta ctatg 25
<210> 2
   <211> 23
   <212> DNA
   <213> Homo sapiens
<400> 2
   ctagtaactc agcagcatct cag 23
<210> 3
   <211> 32
   <212> DNA
   <213> Homo sapiens
<400> 3
   ctactgtttt cctttactta ctacacctca ga 32
<210> 4
   <211> 24
   <212> DNA
   <213> Homo sapiens
<400> 4
   atccagacaa ctgttcaaac tgat 24
<210> 5
   <211> 32
   <212> DNA
   <213> Homo sapiens
<400> 5
   ctactgtttt cctttactta ctacacctca ga 32
<210> 6
   <211> 24
   <212> DNA
   <213> Homo sapiens
<400> 6
   atccagacaa ctgttcaaac tgat 24
<210> 7
   <211> 23
   <212> DNA
   <213> Homo sapiens
<400> 7
   ggtgaaacct gtttgttgga cat 23
<210> 8
   <211> 24
   <212> DNA
   <213> Homo sapiens
<400> 8
   tgtattggtc tctcatggca ctgt 24
<210> 9
   <211> 21
   <212> DNA
   <213> Homo sapiens
<400> 9
   gataggcaga aatgggcttg a 21
<210> 10
   <211> 25
   <212> DNA
   <213> Homo sapiens
<400> 10
   atcatccttt cagagaaaat aatgc 25
<210> 11
   <211> 23
   <212> DNA
   <213> Homo sapiens
<400> 11
   ggtgaaacct gtttgttgga cat 23
<210> 12
   <211> 24
   <212> DNA
   <213> Homo sapiens
<400> 12
   tgtattggtc tctcatggca ctgt 24
<210> 13
   <211> 766
   <212> PRT
   <213> Homo sapiens
<400> 13
<210> 14
   <211> 189
   <212> PRT
   <213> Homo sapiens
<400> 14
   Met Thr Glu Tyr Lys Leu Val Val Val Gly Ala Gly Gly Val Gly Lys
   1               5                   10                  15
   Ser Ala Leu Thr Ile Gln Leu Ile Gln Asn His Phe Val Asp Glu Tyr
               20                  25                  30
   Asp Pro Thr Ile Glu Asp Ser Tyr Arg Lys Gln Val Val Ile Asp Gly
           35                  40                  45
   Glu Thr Cys Leu Leu Asp Ile Leu Asp Thr Ala Gly Gln Glu Glu Tyr
       50                  55                  60
   Ser Ala Met Arg Asp Gln Tyr Met Arg Thr Gly Glu Gly Phe Leu Cys
   65                  70                  75                  80
   Val Phe Ala Ile Asn Asn Ser Lys Ser Phe Ala Asp Ile Asn Leu Tyr
                   85                  90                  95
   Arg Glu Gln Ile Lys Arg Val Lys Asp Ser Asp Asp Val Pro Met Val
               100                 105                 110
   Leu Val Gly Asn Lys Cys Asp Leu Pro Thr Arg Thr Val Asp Thr Lys
           115                 120                 125
   Gln Ala His Glu Leu Ala Lys Ser Tyr Gly Ile Pro Phe Ile Glu Thr
       130                 135                 140
   Ser Ala Lys Thr Arg Gln Gly Val Glu Asp Ala Phe Tyr Thr Leu Val
   145                 150                 155                 160
   Arg Glu Ile Arg Gln Tyr Arg Met Lys Lys Leu Asn Ser Ser Asp Asp
                   165                 170                 175
   Gly Thr Gln Gly Cys Met Gly Leu Pro Cys Val Val Met
               180                 185
<210> 15
   <211> 16
   <212> DNA
   <213> Homo sapiens
<400> 15
   tagctacaga gaaatc 16
<210> 16
   <211> 14
   <212> DNA
   <213> Homo sapiens
<400> 16
   cagctggaaa agaa 14
<210> 17
   <211> 25
   <212> DNA
   <213> Homo sapiens
<400> 17
   ctaagaggaa agatgaagta ctatg 25
<210> 18
   <211> 23
   <212> DNA
   <213> Homo sapiens
<400> 18
   ctagtaactc agcagcatct cag 23
<210> 19
   <211> 21
   <212> DNA
   <213> Homo sapiens
<400> 19
   gataggcaga aatgggcttg a 21
<210> 20
   <211> 25
   <212> DNA
   <213> Homo sapiens
<400> 20
   atcatccttt cagagaaaat aatgc 25
<210> 21
   <211> 19
   <212> DNA
   <213> Homo sapiens
<400> 21
   gaaggtgaag ttcggagtc 19
<210> 22
   <211> 20
   <212> DNA
   <213> Homo sapiens
<400> 22
   gaagatggtg atgggatttc 20
<210> 23
   <211> 21
   <212> DNA
   <213> Homo sapiens
<400> 23
   gaaatggcga tcagcaagac g 21
<210> 24
   <211> 21
   <212> DNA
   <213> Homo sapiens
<400> 24
   cgacgactcg tatagctcct g 21
<210> 25
   <211> 21
   <212> DNA
   <213> Homo sapiens
<400> 25
   atcagatcag agccatccga a 21
<210> 26
   <211> 21
   <212> DNA
   <213> Homo sapiens
<400> 26
   tggagtctct cgtgtttgtg c 21
<210> 27
   <211> 19
   <212> DNA
   <213> Homo sapiens
<400> 27
   ggtcagtggc ctagtgagc 19
<210> 28
   <211> 19
   <212> DNA
   <213> Homo sapiens
<400> 28
   tgctgtcgtt ggatggcac 19